# EUROPEAN PATENT APPLICATION

(11) **EP 4 212 163 A1**
(43) Date of publication of application: **19.07.2023**
(21) Application number: 21866525.5
(22) Date of filing: 24.08.2021
(51) Int. Cl.: A61K 35/32, A61K 31/56, A61K 35/28, A61K 35/51, A61K 38/44, A61K 39/395, A61P 11/00, A61P 29/00, A61P 37/06, A61P 43/00

(54) **CYTOKINE STORM INHIBITOR, METHOD FOR USING CYTOKINE STORM INHIBITOR, AND METHOD FOR SCREENING FOR CYTOKINE INHIBITOR**

(30) Priority: 08.09.2020 JP 2020150775; 29.07.2021 JP 2021124104
(71) Applicant: Dexon Pharmaceuticals Inc., Tokyo, 103-0027 (JP)
(72) Inventor: KOGA Shoji, Tokyo 103-0027 (JP)
(74) Representative: Godemeyer Blum Lenze Patentanwälte Partnerschaft mbB - werkpatent
(86) International application number: PCT/JP2021/030978
(87) International publication number: WO 2022/054565

(57) **Abstract**

A cytokine storm suppressor containing microparticles derived from a culture supernatant of dental pulp-derived stem cells, adipose-derived stem cells, bone marrow-derived stem cells, umbilical cord-derived stem cells or immortalized stem cells thereof in which the microparticles contain extracellular superoxide dismutase (SOD3) in an effective amount capable of repairing a tissue or a cell damaged by a cytokine storm or a higher amount and which is used for administration to a human or a nonhuman animal with a cytokine storm before damage of a tissue is caused by the cytokine storm to suppress the tissue damage by the cytokine storm through suppression of the cytokine storm or used for administration to a human or a nonhuman animal with damage of a tissue caused by a cytokine storm to repair the tissue damaged by the cytokine storm through suppression of the cytokine storm; a method for using the cytokine storm suppressor; and a screening method.

## Description

### Technical Field

The present invention relates to a cytokine storm suppressor, a method for using a cytokine storm suppressor and a screening method.

### Background Art

### <Cytokine Storm>

Cytokine is a general name for proteins involved in inflammatory reaction and immune response, and several hundred kinds or more are known. Cytokines each have various activities, and well-balanced, coordinated interactions thereof maintain or regulates biological functions. Cytokines are generally produced/released through immune response to infection or the like. However, the blood concentrations of two or more cytokine kinds sometimes increase abnormally due to uncontrolled production/release of the cytokines for some reasons, and as a result, tissues in the body are sometimes damaged. The state with an abnormally increased blood cytokine concentration is called a cytokine storm (hypercytokinemia).

Known causes for a cytokine storm or diseases causing a cytokine storm include infection, drug administration, invasion of highly advanced surgery, sepsis, systemic inflammatory response syndrome (SIRS), disseminated intravascular coagulation (DIC), multiple organ failure and the like. Of these, the most common cause is sepsis, and it has been recently known that viremia, especially COVID-19, causes a cytokine storm (NPL 1; Inflammation and Regeneration (2020) 40:14).

In animal experiment, administration of lipopolysaccharide (LPS, endotoxin) can cause symptoms of sepsis such as an increased inflammatory cytokine concentration and induce a cytokine storm (NPL 2: Medical Hypotheses (2020) 144,109865).

### <Problems of Conventional Immunosuppressive Agents>

As a method for suppressing a cytokine storm, a method for suppressing the amount of a cytokine such as an inflammatory cytokine using an immunosuppressive agent such as anti-interleukin (IL)-6 antibodies including tocilizumab and steroids is widely known. For example, NPL 3 (Nature communications (2016) 7:11498) describes that, when an anti-IL-6 antibody was administered to mice to which LPS was administered, the survival rate was 75% under the conditions where the mice to which the control antibody was administered all died 96 hours after the LPS administration.

The method for suppressing an inflammatory cytokine with an immunosuppressive agent such as anti-IL-6 antibodies and steroids, or the like, however, is not sufficient to suppress a cytokine storm and has a problem because a (neighboring) tissue in which a cytokine storm is caused cannot be repaired. Thus, it is believed recently that, even when a cytokine storm is treated with an immunosuppressive agent, an aftereffect derived from the cytokine storm remains.

### Citation List

### Patent Literature

[PTL 1] JP6327647B

### Non Patent Literature

[NPL 1] Inflammation and Regeneration (2020) 40:14
[NPL 2] Medical Hypotheses (2020) 144,109865
[NPL 3] Nature communications (2016) 7:11498
[NPL 4] Stem Cell Rev and Rep (2020) 6:548-559)
[NPL 5] Cell Biosci (2020) 10:22
[NPL 6] ANTIOXIDANTS & REDOX SIGNALING (2020), 33, 2
[NPL 7] STEM CELLS AND DEVEROPMENT (2020), 29, 12, 747-754
[NPL 8] Cytotherapy 00 (May 2, 2000) 1-4
[NPL 9] Cells (2019) 8, 1605
[NPL 10] Front. Bioeng. Biotechnol. (2020), 8:554
[NPL 11] Cells (2019) 8, 1240
[NPL 12] J. Neurochem. (2010) 114, 1569-1580
[NPL 13] Mol Genet Genomic Med. (2019) 7:e831

### Summary of Invention

### Technical Problem

Here, it is known that a composition containing a culture supernatant of mesenchymal stem cells, namely unisolated microparticles such as exosomes, suppresses cytokine-related inflammation. For example, PTL 1 describes a composition for preventing or treating an inflammatory disease containing a culture supernatant obtained by culturing dental pulp stem cells, and the inflammatory disease is selected from the group consisting of fulminant hepatitis, acute hepatitis, chronic hepatitis, hepatic cirrhosis, acute interstitial pneumonia, chronic interstitial pneumonia, pulmonary fibrosis, an inflammatory autoimmune disease and an ischemic heart disease. PTL 1 discloses, in [0033], systemic inflammations such as shock induced by chemotherapy methods that respond to proinflammatory cytokines (for example, proinflammatory cytokine-associated shock) as the inflammatory disease. PTL 1, however, has not examined the exosomes of mesenchymal stem cells.

A problem that the invention is to solve is to provide a cytokine storm suppressor which can suppress multiple cytokines involved in a cytokine storm and which can suppress tissue damage by a cytokine storm or repair a tissue damaged by a cytokine storm.

### Solution to Problem

### <Relations between SOD3 and Cytokine Storm>

It is known that extracellular superoxide dismutase (SOD3) weakens damage of a tissue and reduces inflammation in the presence of IFNγ and TNF (NPL 4; Stem Cell Rev and Rep (2020) 6:548-559).

MSCs are known to secrete SOD3, and the SOD3 secreted from MSCs is known to be capable of repairing a tissue through an antioxidative effect and immunoregulation (NPL 5; Cell Biosci (2020) 10:22). However, exosomes are not disclosed.

It is suggested that the low SOD3 state of the lungs of the elderly is linked to the severity of COVID-19 derived from a cytokine storm (NPL 6; ANTIOXIDANTS & REDOX SIGNALING (2020), 33, 2).

### <Relations between MSC Exosomes and Cytokine Storm (No Suggestion about SOD3)>

Although it is known to use MSC exosomes to suppress a cytokine storm, it is not known that SOD3 is involved in the cytokine suppression of MSC exosomes.

For example, although NPL 2 (Medical Hypotheses (2020) 144,109865) discloses, in the abstract and the right column on page 2, that mesenchymal stem cells (MSCs) isolated from adipose, bone marrow or an umbilical cord or MSC exosomes isolated from a culture supernatant thereof suppress inflammatory cytokines and discloses the effect of repairing a tissue damaged by a cytokine storm, SOD3 is not disclosed.

Although a plan for suppressing and treating a cytokine storm with MSC exosomes is described in NPL 1 (Inflammation and Regeneration (2020) 40:14), from the right column on page 3 to the left column on page 4, SOD3 is not disclosed.

Although downregulation of a cytokine storm by bone marrow-derived MSC exosomes is described in NPL 7 (STEM CELLS AND DEVEROPMENT (2020), 29, 12, 747-754), SOD3 is not written.

Although it is known that a cytokine storm is involved in COVID-19 pneumonia and that a method using extracellular vesicles (exosomes) of mesenchymal stem cells (MSCs) is effective for treating COVID-19 infection (NPL 8; Cytotherapy 00 (May 2, 2000) 1-4), SOD3 is not disclosed.

Although NPL 9 (Cells (2019) 8, 1605) discloses that MSC exosomes activate autophagy, suppress apoptosis, necrosis and oxidative stress in an injured tissue and promote regeneration through the delivery of mRNAs and miRNAs, as the effect of suppressing inflammation induced by LPS administration, SOD3 is not disclosed.

Although NPL 10 (Front. Bioeng. Biotechnol. (2020), 8:554.) discloses that cells and tissues in a cytokine storm state are under oxidative stress and that administration of MSCs and exosomes can suppress inflammation and oxidative stress and can also regenerate lungs, SOD3 is not disclosed.

### <Tissue Repair by MSC Exosomes (No Suggestion about either SOD3 or Cytokine Storm)>

Although NPL 11 (Cells (2019) 8, 1240) discloses that MSC exosomes regenerate damaged kidneys, SOD3 and a cytokine storm are not disclosed.

<Relations between MSCs and SOD3 (No Suggestion about either Exosomes or Cytokine Storm)>

Although NPL 12 (J. Neurochem. (2010) 114, 1569-1580) discloses that SOD3 secreted from bone marrow-derived MSCs protects neurons, exosomes are not disclosed.

Although NPL 13 (Mol Genet Genomic Med. (2019) 7:e831) discloses that MSCs which highly express SOD3 suppress ischemic stroke, exosomes are not disclosed.

### <New Findings in Invention>

The present inventors have found that microparticles such as exosomes derived from a culture supernatant of specific mesenchymal stem cells contain a large amount of extracellular superoxide dismutase (SOD3) and exhibit a high SOD activity.

The inventors have also found a synergistic effect in which the microparticles themselves directly suppress inflammatory cytokines and the like in a cytokine storm induced by LPS administration and in which SOD3 contained in the microparticles promotes repair of a tissue damaged in the presence of IFNγ and TNFα, of cytokines, or suppresses tissue damage by a cytokine storm when it is before damage of a tissue is caused.

### <Differences in Mechanism of Action from Conventional Cytokine Storm Suppressors and Applications to Elderly>

It has been known so far that SOD3 protein exists in exosomes. However, that the SOD3 activity of exosomes derived from a culture supernatant of specific mesenchymal stem cells is very high and that SOD3 is contained in an effective amount capable of repairing a tissue damaged by a cytokine storm or a higher amount are findings that the present inventors have made for the first time. Without the findings, a cytokine storm suppressor which can suppress cytokines involved in a cytokine storm and which can suppress tissue damage by a cytokine storm or repair a tissue damaged by a cytokine storm cannot be achieved, and thus the cytokine storm suppressor of the invention has a totally different mechanism of action from those of the conventional cytokine storm suppressors.

Furthermore, it is expected that tissues of elderly people with a low SOD3 level (NPL 6; ANTIOXIDANTS & REDOX SIGNALING (2020), 33, 2) cannot be repaired only by suppressing cytokines because the SOD3 level is low. According to the invention, however, a tissue of the elderly damaged by a cytokine storm can be repaired significantly, or tissue damage by a cytokine storm can be suppressed significantly when it is before the damage of a tissue is caused.

As a result of intensive studies to solve the problem, the inventors have found that microparticles such as exosomes derived from a culture supernatant of specific mesenchymal stem cells contain extracellular superoxide dismutase (SOD3) and exhibit a high SOD activity. The inventors have also found that multiple cytokines involved in a cytokine storm can be suppressed significantly using the microparticles.

Specifically, the invention and preferable constitution of the invention are as follows.
[1] A cytokine storm suppressor containing microparticles derived from a culture supernatant of dental pulp-derived stem cells, adipose-derived stem cells, bone marrow-derived stem cells, umbilical cord-derived stem cells or immortalized stem cells thereof,
   in which the cytokine storm suppressor is not the culture supernatant, does not contain the dental pulp-derived stem cells, the adipose-derived stem cells, the bone marrow-derived stem cells, the umbilical cord-derived stem cells or the immortalized stem cells thereof, does not contain MCP-1 and does not contain Siglec-9, and
   the cytokine storm suppressor is used for administration to a human or a nonhuman animal with a cytokine storm before damage of a tissue is caused by the cytokine storm to suppress the tissue damage by the cytokine storm through suppression of the cytokine storm or
   used for administration to a human or a nonhuman animal with damage of a tissue caused by a cytokine storm to repair the tissue damaged by the cytokine storm through suppression of the cytokine storm.
   [1-1] A cytokine storm suppressor containing microparticles derived from a culture supernatant of dental pulp-derived stem cells, adipose-derived stem cells, bone marrow-derived stem cells, umbilical cord-derived stem cells or immortalized stem cells thereof,
   in which the microparticles contain extracellular superoxide dismutase (SOD3) in an effective amount capable of repairing a tissue or a cell damaged by a cytokine storm or a higher amount, and
   the cytokine storm suppressor repairs a tissue damaged by a cytokine storm through suppression of the cytokine storm.
[2] The cytokine storm suppressor described in [1] or [1-1], in which the microparticles are exosomes.
[3] The cytokine storm suppressor described in [1], [1-1] or [2], in which the microparticles are microparticles purified from the culture supernatant.
[4] The cytokine storm suppressor described in any one of [1] to [3] (including [1-1], the same applies below) which suppresses all of IL-2, IL-4, IL-6, IL-10, IL-17, IFNγ and TNFα in a cell or in blood.
[5] The cytokine storm suppressor described in any one of [1] to [4] which increases the survival rate when the cytokine storm suppressor is administered to a human or a nonhuman animal in a cytokine storm state compared to when the cytokine storm suppressor is not administered.
[6] The cytokine storm suppressor described in any one of [1] to [5], in which the microparticles contain 1.0 ng/mg or more of the SOD3 per 1 mg of the microparticles.
[7] The cytokine storm suppressor described in any one of [1] to [6] which has a SOD3 activity of 0.3 unit/µg or more.
[8] The cytokine storm suppressor described in any one of [1] to [7] which contains 0.01×10⁸ particles/ml or more of the microparticles.
[9] The cytokine storm suppressor described in any one of [1] to [7] which contains 2.0×10⁹ particles/ml or more of the microparticles.
[10] The cytokine storm suppressor described in any one of [1] to [9], in which the dental pulp-derived stem cells, the adipose-derived stem cells, the bone marrow-derived stem cells or the umbilical cord-derived stem cells are human dental pulp-derived stem cells, human adipose-derived stem cells, human bone marrow-derived stem cells or human umbilical cord-derived stem cells.
[11] The cytokine storm suppressor described in any one of [1] to [10], in which the microparticles are microparticles derived from a culture supernatant of the dental pulp-derived stem cells.
[12] The cytokine storm suppressor described in any one of [1] to [11], in which the tissue damaged by the cytokine storm is a lung tissue.
[13] The cytokine storm suppressor described in any one of [1] to [12], in which the dental pulp-derived stem cells, the adipose-derived stem cells, the bone marrow-derived stem cells or the umbilical cord-derived stem cells are genetically modified cells in which the microparticles highly express the SOD3.
[14] The cytokine storm suppressor described in any one of [1] to [13] which contains at least one of an anti-IL-6 antibody and a steroid.
[15] The cytokine storm suppressor described in any one of [1] to [14] which is used for administration to a human or a nonhuman animal with a cytokine storm before damage of a tissue is caused by the cytokine storm to suppress the tissue damage by the cytokine storm through suppression of the cytokine storm and repair a tissue to be damaged by the cytokine storm.
[16] The cytokine storm suppressor described in any one of [1] to [15], in which the cytokine storm suppressor is a tissue-repairing agent.
[16-1] The cytokine storm suppressor described in any one of [1] to [16], in which the cytokine storm suppressor does not contain the culture supernatant from which the microparticles have been excluded.
[17] A method for using a cytokine storm suppressor
   which includes administering the cytokine storm suppressor described in any one of [1] to [16] and [16-1] to a human or a nonhuman animal
   to decrease the cellular or blood cytokine amount of the human or the nonhuman animal and
   which includes
   a step of administering to the human or the nonhuman animal with a cytokine storm before damage of a tissue is caused by the cytokine storm to suppress the tissue damage by the cytokine storm through suppression of the cytokine storm or
   a step of administering to the human or the nonhuman animal with damage of a tissue caused by a cytokine storm to repair the tissue damaged by the cytokine storm through suppression of the cytokine storm.
[18] The method for using a cytokine storm suppressor described in [17], in which the cytokine storm suppressor is used in combination with at least one of an anti-IL-6 antibody and a steroid.
[19] A screening method for selecting a factor or a combination of factors which is effective for tissue repair for repairing a tissue damaged by a cytokine storm through suppression of the cytokine storm, having
   a step of supplying one, two or more components contained in a culture supernatant obtained by culturing dental pulp-derived stem cells, adipose-derived stem cells, bone marrow-derived stem cells, umbilical cord-derived stem cells or immortalized stem cells thereof to an evaluation system related to extracellular superoxide dismutase (SOD3) and evaluating a SOD3-related feature.
[20] A cytokine storm suppressor containing a component obtained by evaluation of the SOD3-related feature by the screening method described in [19],
   in which the component is derived from a culture supernatant of dental pulp-derived stem cells, adipose-derived stem cells, bone marrow-derived stem cells, umbilical cord-derived stem cells or immortalized stem cells thereof,
   the component contains extracellular superoxide dismutase (SOD3) in an effective amount capable of repairing a tissue or a cell damaged by a cytokine storm or a higher amount,
   the cytokine storm suppressor is not the culture supernatant, does not contain the dental pulp-derived stem cells, the adipose-derived stem cells, the bone marrow-derived stem cells, the umbilical cord-derived stem cells or the immortalized stem cells thereof, does not contain MCP-1 and does not contain Siglec-9,
   the component satisfies at least one of the feature that 1.0 ng/mg or more of the SOD3 is contained per 1 mg of the component and the feature that the SOD3 activity is 0.3 unit/µg or more, and
   the cytokine storm suppressor is used for administration to a human or a nonhuman animal with a cytokine storm before damage of a tissue is caused by the cytokine storm to suppress the tissue damage by the cytokine storm through suppression of the cytokine storm or
   used for administration to a human or a nonhuman animal with damage of a tissue caused by a cytokine storm to repair the tissue damaged by the cytokine storm through suppression of the cytokine storm.

### Advantageous Effects of Invention

According to the invention, a cytokine storm suppressor which can suppress multiple cytokines involved in a cytokine storm and which can suppress tissue damage by a cytokine storm or repair a tissue damaged by a cytokine storm can be provided.

### Brief Description of Drawings

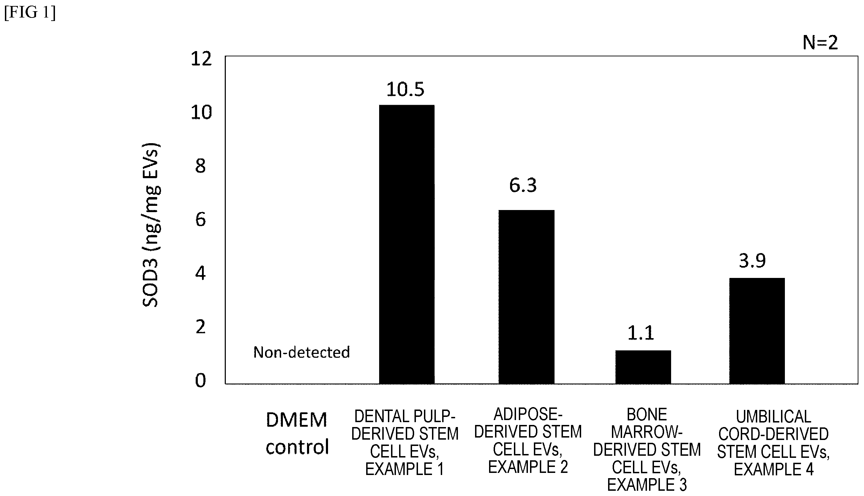

[Fig. 1] Fig. 1 is a graph showing the SOD3 protein amounts in the cytokine storm suppressors of the Examples or DMEM medium used as a control.

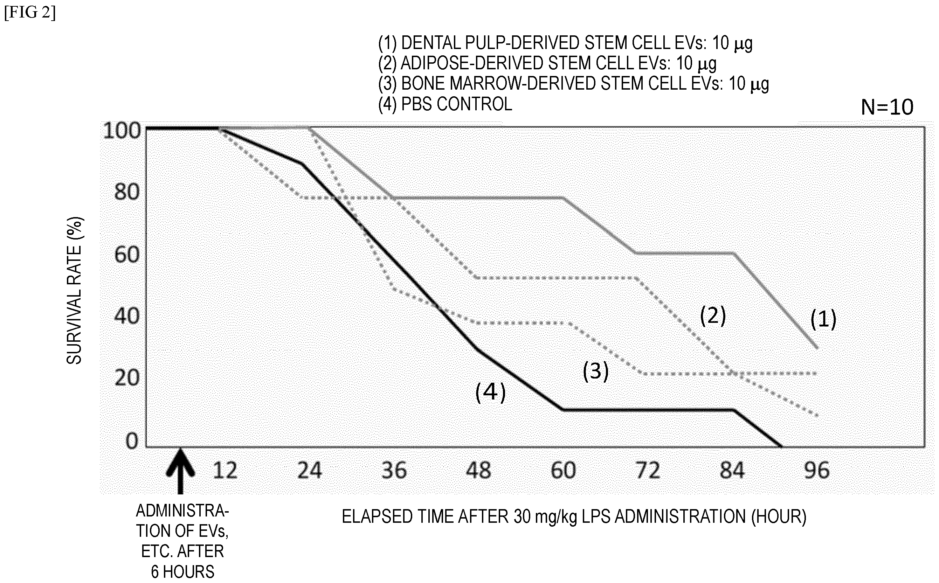

[Fig. 2] Fig. 2 is a graph showing the relations between the time after the LPS administration and the survival rate, where the cytokine storm suppressors of the Examples or physiological saline (PBS control) used as a control was administered once to mice to which lipopolysaccharide (LPS) was administered.

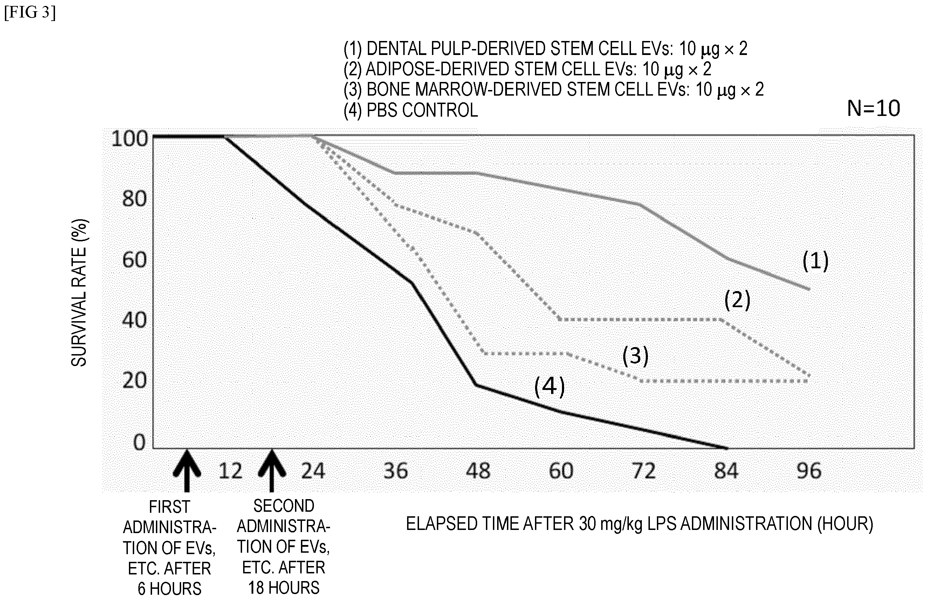

[Fig. 3] Fig. 3 is a graph showing the relations between the time after the LPS administration and the survival rate, where the cytokine storm suppressors of the Examples or the PBS control was administered twice to mice to which LPS was administered.

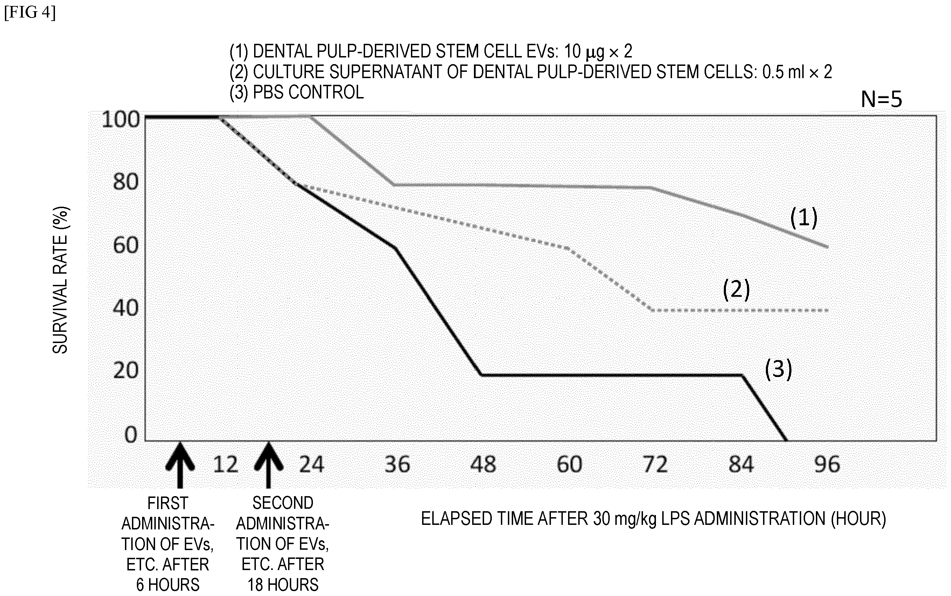

[Fig. 4] Fig. 4 is a graph showing the relations between the time after the LPS administration and the survival rate, where the cytokine storm suppressor of Example 1, a culture supernatant of dental pulp-derived stem cells or the PBS control was administered twice to mice to which LPS was administered.

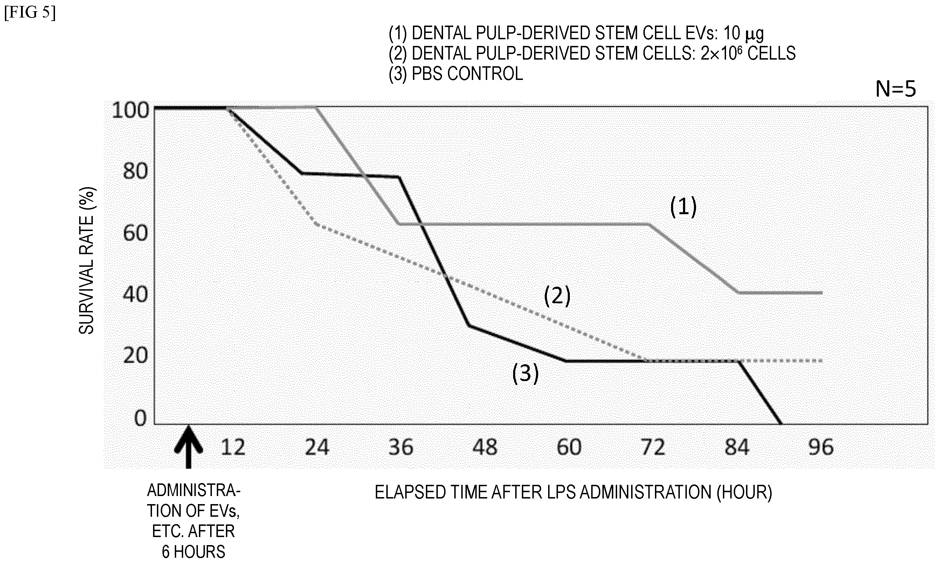

[Fig. 5] Fig. 5 is a graph showing the relations between the time after the LPS administration and the survival rate, where the cytokine storm suppressor of Example 1, dental pulp-derived stem cells or the PBS control was administered to mice to which LPS was administered.

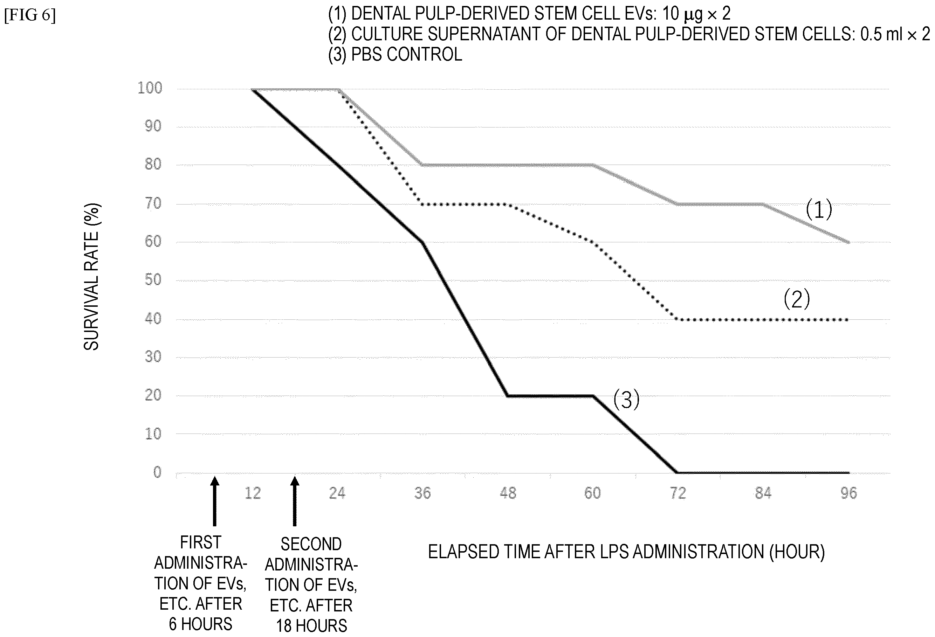

[Fig. 6] Fig. 6 is a graph showing the relations between the time after the LPS administration and the survival rate, where the cytokine storm suppressor of Example 1, a culture supernatant of dental pulp-derived stem cells or the PBS control was administered twice, namely six hours and 18 hours after the LPS administration, to mice to which LPS was administered.

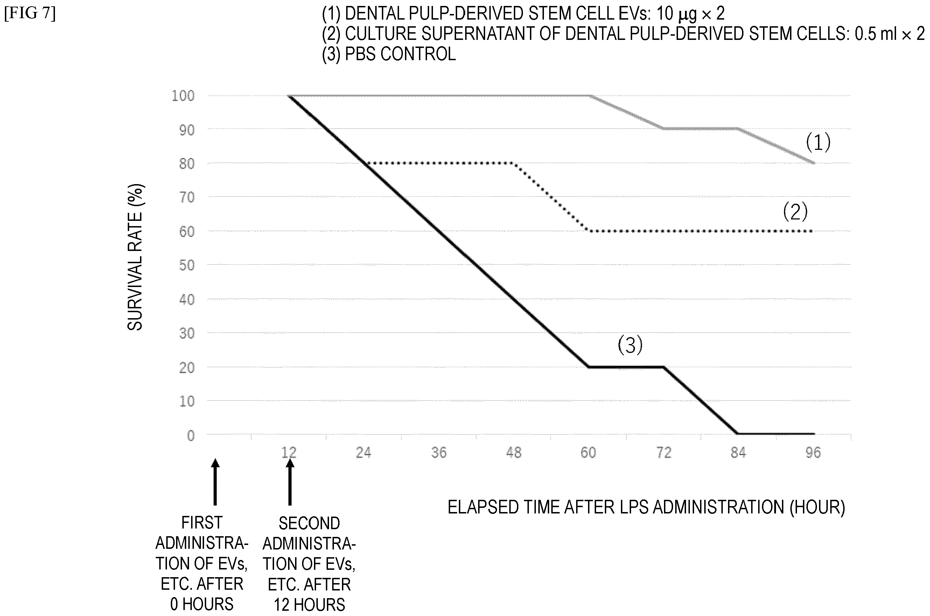

[Fig. 7] Fig. 7 is a graph showing the relations between the time after the LPS administration and the survival rate, where the cytokine storm suppressor of Example 1, a culture supernatant of dental pulp-derived stem cells or the PBS control was administered twice, namely after 0 hour and after 12 hours, to mice to which LPS was administered.

### Description of Embodiments

The invention is explained in detail below. The constituent features described below are sometimes explained based on typical embodiments or specific examples, but the invention should not be limited to the embodiments. In the present description, a numerical range expressed using "to" means a range including the values before and after "to" as the lower limit and the upper limit.

### [Cytokine Storm Suppressor]

The cytokine storm suppressor of the invention contains microparticles derived from a culture supernatant of dental pulp-derived stem cells, adipose-derived stem cells, bone marrow-derived stem cells, umbilical cord-derived stem cells or immortalized stem cells thereof. The microparticles contain extracellular superoxide dismutase (SOD3) in an effective amount capable of repairing a tissue or a cell damaged by a cytokine storm or a higher amount, and the cytokine storm suppressor is used for administration to a human or a nonhuman animal with a cytokine storm before damage of a tissue is caused by the cytokine storm to suppress the tissue damage by the cytokine storm through suppression of the cytokine storm or used for administration to a human or a nonhuman animal with damage of a tissue caused by a cytokine storm to repair the tissue damaged by the cytokine storm through suppression of the cytokine storm.

The cytokine storm suppressor of the invention can suppress multiple cytokines involved in a cytokine storm and can suppress tissue damage by a cytokine storm or repair a tissue damaged by a cytokine storm.

Preferable aspects of the cytokine storm suppressor of the invention are explained below.

The cytokine storm suppressor of the invention is used for administration to a human or a nonhuman animal with a cytokine storm before damage of a tissue is caused by the cytokine storm to suppress the tissue damage by the cytokine storm through suppression of the cytokine storm or used for administration to a human or a nonhuman animal with damage of a tissue caused by a cytokine storm to repair the tissue damaged by the cytokine storm through suppression of the cytokine storm.

The cytokine storm suppressor of the invention is preferably a tissue damage suppressor, more preferably a tissue-repairing agent.

The repair of a damaged tissue includes regeneration of the tissue, partial regeneration, repair with cicatrix or protection.

The regeneration of a tissue means that a damaged tissue is restored to a degree morphologically and functionally equivalent to the original tissue. In this case, the lack of the damaged tissue is preferably filled with the same cells as the cells constituting the original tissue.

The partial regeneration of a tissue means that a damaged tissue is restored to some extent, but not to a degree morphologically and functionally equivalent to the original tissue. In this case, the lack of the damaged tissue is also preferably filled with the same cells as the cells constituting the original tissue.

The repair of a tissue with cicatrix means that a damaged tissue is restored with remaining cicatrix (substitution with granulation cells or collagen fibers). In this case, the damaged tissue may be restored to a degree either morphologically or functionally equivalent to the original tissue.

The protection of a tissue means that a further increase in the degree of damage of a damaged tissue is suppressed.

Of these, the cytokine storm suppressor of the invention can preferably suppress, regenerate or partially regenerate damage of a tissue.

In the invention, because the microparticles contain extracellular superoxide dismutase (SOD3) in an effective amount capable of repairing a tissue or a cell damaged by a cytokine storm or a higher amount, the cytokine storm suppressor of the invention preferably has a SOD3 activity. The cytokine storm suppressor of the invention preferably contains 1.0 ng/mg or more of SOD3, more preferably contains 3.0 ng/mg or more, particularly preferably contains 6.0 ng/mg or more and further particularly preferably contains 10.0 ng/mg or more. In a preferable aspect of the cytokine storm suppressor of the invention, the SOD3 activity is further improved because SOD3 is contained preferably at a high concentration. The cytokine storm suppressor of the invention preferably has a SOD3 activity of 0.3 unit/µg or more, further preferably 0.4 unit/µg or more, more preferably 1.0 unit/µg or more, particularly preferably 3.0 unit/µg or more, further particularly preferably 4.0 unit/µg or more.

When the microparticles do not contain extracellular superoxide dismutase (SOD3) in an effective amount capable of repairing a tissue or a cell damaged by a cytokine storm or a higher amount, it is difficult to increase the SOD3 activity. In a preferable aspect of the cytokine storm suppressor of the invention, the SOD3 activity is further improved in this manner because specific microparticles containing sufficient SOD3 are contained at a high concentration.

### <Tissue Damaged by Cytokine Storm>

The kind of the tissue which is damaged by a cytokine storm and which can be inhibited from being damaged or can be repaired with the cytokine storm suppressor of the invention is not particularly restricted and may be any tissue. The tissue which is damaged by a cytokine storm and which can be inhibited from being damaged or can be repaired with the cytokine storm suppressor of the invention is preferably any of all organs, tracheae, nerves and vessels, more preferably a lung tissue, a cardiac tissue, a hepatic tissue, a gallbladder tissue, a spleen tissue, a renal tissue, an esophageal tissue, a stomach tissue, a small intestine tissue, a large intestine tissue, a rectal tissue, a bladder tissue, a tracheal tissue, a neural tissue, a thyroid tissue or a vascular tissue, particularly preferably a lung tissue, a tracheal tissue or a vascular tissue, further particularly preferably a lung tissue.

### <Suppression of Cytokine Storm>

### (Explanation of Types of Cytokines)

When the tissue-repairing agent of the invention suppresses tissue damage by a cytokine storm or repairs a tissue damaged by a cytokine storm through suppression of the cytokine storm, cytokines are suppressed. The suppressed cytokines are not particularly restricted. Examples of the suppressed cytokines include IL-2, IL-4, IL-6, IL-10, IL-17, IFNγ, TNFα and the like. In addition, other suppressed cytokines include IL-1, IL-18, MIG, MIP-1α and the like.

IL-1 is an inflammatory cytokine involved in inflammation and protection against infection.

IL-2 is a cytokine involved in cellular immunity. Excessive expression of IL-2 leads to a selective increase in regulatory T cells (Tregs), which are a subset of T cells. Tregs exhibit an action of maintaining peripheral tolerance by suppressing immune response of other cells. Breakdown of peripheral tolerance is believed to cause autoimmune diseases in humans. Thus, the immunosuppressive ability of Tregs is believed to prevent the development of autoimmune diseases. Moreover, Tregs are involved also in cancer, and solid tumors and hematologic malignancies are associated with elevated numbers of Tregs (see [0007] of JP2020-002154A).

IL-4 is a non-redundant cytokine involved in the differentiation of T helper cells into the Th2 (T helper type 2) subset, and Th2 promotes the differentiation of premature B cells into IgE-producing plasma cells. The IgE level is elevated in allergic asthma. Thus, IL-4 is involved in the development of allergic asthma (see [0008] of JP2020-002154A).

IL-6 is a B cell differentiation factor, a B cell-stimulating factor-2, a hepatocyte-stimulating factor, a hybridoma growth factor and a plasmacytoma growth factor. IL-6 is a typical inflammatory cytokine and is a multifunctional cytokine involved in regulation of acute inflammatory response, modulation of specific immune responses including B and T cell differentiation, bone metabolism, thrombopoiesis, epidermal proliferation, menses, neuronal cell differentiation, neuroprotection, aging, cancer, inflammatory reaction occurring in Alzheimer's disease and the like. IL-6 is believed to play a role in the development of many diseases and disorders, including fatigue, cachexia, autoimmune diseases, diseases of the skeletal system, cancer, heart disease, obesity, diabetes, asthma, Alzheimer's disease and multiple sclerosis (see [0002] to [0005] of JP2019-047787A).

IL-10 is a homodimer protein having 160 amino acid residues produced in T cells, macrophages and dendric cells. IL-10 is known to be involved in suppression of macrophage functions, B cell activation and the like (see [0052] of WO2018/212237A1). Moreover, IL-10 is an anti-inflammatory cytokine.

IL-17 is a protein having 132 amino acid residues produced in CD4 memory T cells and Th17 cells. IL-17 is known to be involved in inflammatory cytokine production from macrophages, epithelial cells, endothelial cells and fibroblasts and the like (see [0053] of WO2018/212237A1).

IL-18 is released when a severe stress is applied to cells due to viral infection, cancer reactive oxygen species or the like.

IFNs (interferons) have the functions of inhibiting viral replication and activating immune cells (for example, natural killer cells, macrophages and the like) and the like. IFNs are divided into type I IFNs, type II IFNs and type III IFNs. Of these, a type II IFN, IFNγ, is a pleiotropic cytokine which is produced by activated immune cells and can induce cellular responses such as an increase in macrophage activity, an increase in the expression of MHC molecules and an increase in NK cell activity (see [0209] of JP2020-522254A).

TNF (tumor necrosis factor) is a typical inflammatory cytokine. TNFα, TNFβ and LTβ are known as TNFs. Of these, TNFα mediates a number of important vital functions, including structural and functional organization of secondary lymphoid organs, apoptosis and antitumor activity, inhibition of viral replication, immunoregulation and inflammation. TNFs also play important roles in pathogenesis of autoimmune diseases, acute phase reaction, septic shock, fever and cachexia (see [0004] of JP2020-079306A).

MIG is a cytokine which governs type 1 immune response. MIG is produced by macrophages, epithelial cells, vascular endothelial cells and the like and causes Th1 cells, CD8T cells, some macrophages and the like to migrate to an inflammation site.

MIP-1α is a cytokine produced by macrophages, fibroblasts, epithelial cells, vascular smooth muscle cells and the like in response to inflammation.

Of the cytokines, IFNγ and TNFα are important. IFNγ and TNFα which exist in the case of a cytokine storm cooperate with SOD3 of exosomes (see Stem Cell Rev and Rep (2020) 6:548-559) and can repair tissue damage caused by the cytokine storm. It is preferable that IFNγ and TNFα are not suppressed completely and remain to some extent because tissue damage caused by the cytokine storm can be easily repaired.

The cytokine storm suppressor of the invention preferably suppresses at least two types of cytokines of the group consisting of IL (interleukin)-2, IL-4, IL-6, IL-10, IL-17, IFNγ and TNFα in a cell or in blood and more preferably suppresses at least four types of cytokines of the group. The cytokine storm suppressor of the invention particularly preferably suppresses all of IL-2, IL-6, IL-10, IL-17, IFNγ and TNFα and further particularly preferably suppresses all of IL-2, IL-4, IL-6, IL-10, IL-17, IFNγ and TNFα.

Moreover, the cytokine storm suppressor of the invention preferably increases the extracellular superoxide dismutase (SOD3) activity when the cytokine storm suppressor is administered to a human or a nonhuman animal in a cytokine storm state.

Furthermore, the cytokine storm suppressor of the invention preferably increases the survival rate when the cytokine storm suppressor is administered to a human or a nonhuman animal in a cytokine storm state compared to when the cytokine storm suppressor is not administered.

### <Uses>

The cytokine storm suppressor of the invention can be used as a therapeutic medicine or a prophylactic medicine for a cytokine storm-related disease, such as infection, drug administration, invasion of highly advanced surgery, sepsis, systemic inflammatory response syndrome (SIRS), disseminated intravascular coagulation (DIC), multiple organ failure and other inflammation, or as a therapeutic medicine or a prophylactic medicine for an aftereffect of a cytokine storm. The SOD3 contained in the microparticles contained in the cytokine storm suppressor of the invention can suppress (downregulate) multiple cytokines involved in a cytokine storm and can cooperate with IFNγ and TNFα to repair tissue damage caused by the cytokine storm. Thus, the cytokine storm suppressor can be used as a therapeutic medicine or a prophylactic medicine for infection with a coronavirus (COVID-19 or the like). In fact, it is suggested that the low SOD3 state in the lungs of the elderly is linked to the severity of COVID-19 (ANTIOXIDANTS & REDOX SIGNALING (2020), 33, 2). Moreover, cells in a cytokine storm state are suggested to be under oxidative stress (Front. Bioeng. Biotechnol. (2020), 8:554), and administration of a large amount of SOD3 with high activity contained in the microparticles contained in the cytokine storm suppressor of the invention can enhance the antioxidative action and suppress the oxidative stress of the cells in a cytokine storm state.

The cytokine storm suppressor of the invention is capable of suppressing a cytokine storm in this manner, preventing or treating a cytokine storm-related disease and increasing the survival rate from a cytokine storm-related disease as well as capable of repairing tissue damage caused by a cytokine storm, and thus the cytokine storm suppressor is used as a medicine for improving an aftereffect of a cytokine storm.

In this regard, however, the cytokine storm suppressor of the invention may be used for use other than the uses. In all the cases, the cytokine storm suppressor of the invention is preferably a pharmaceutical composition.

The cytokine storm suppressor of the invention can be used as a therapeutic medicine or a prophylactic medicine for a virus disease such as COVID-19, of infection and sepsis. Here, when some coronaviruses such as SARS-CoV and SARS-CoV-2, which is the virus that causes COVID-19, infect a human or a nonhuman animal, a cytokine storm state is caused.

Even when pharmacological test results as a therapeutic medicine for COVID-19 may not have been shown when the present application was filed, it can be said that the cytokine storm suppressor of the invention can be used (or is possibly used) as a therapeutic medicine for COVID-19.

Having COVID-19 (being infected with the new coronavirus) means that the living body of a human or a nonhuman animal has cells infected with SARS-CoV-2. The infection can be confirmed especially by a method of detection or viral titration from a respiratory tract sample, a method for detecting a blood-circulating SARS-CoV-2-specific antibody or the like, and a known method using PCR is used at this point.

The prevention of COVID-19 means prevention of infection of the living body of a human or a nonhuman animal with SARS-CoV-2 or at least reduction of the possibility of onset thereof.

The cytokine storm suppressor of the invention may be used as a therapeutic medicine for COVID-19. The treatment of COVID-19 means reducing or weakening a symptom associated with viral infection (respiratory syndrome, fever or the like) in the living body of a human or a nonhuman animal or shortening the recovery period of the symptom. The viral infection rate (infectivity potency) of a human or animal body preferably decreases through administration of the cytokine storm suppressor of the invention, and it is more preferable that the virus disappears completely from the living body.

Furthermore, the cytokine storm suppressor of the invention is used as a prophylactic medicine or a therapeutic medicine for an aftereffect of COVID-19. The cytokine storm suppressor of the invention can repair tissue damage caused by a cytokine storm through suppression of the cytokine storm and thus is used as a prophylactic medicine or a therapeutic medicine for an aftereffect of COVID-19, which is believed to be caused by an aftereffect of a cytokine storm.

Compared to the conventional compositions which can be used as cytokine storm suppressors and immunosuppressive agents, the cytokine storm suppressor of the invention has advantages such as easiness of mass production, utilization of culture solutions of stem cells which are otherwise disposed of as industrial waste or the like and decreased cost of the disposal of culture solutions of stem cells. In particular, when the culture supernatant of dental pulp-derived stem cells or the like is a culture supernatant of human dental pulp-derived stem cells, human adipose-derived stem cells, human bone marrow-derived stem cells or human umbilical cord-derived stem cells, the cytokine storm suppressor of the invention also has advantages of high safety for example from the immunological point of view and less ethical issues in application to a human. When the culture supernatant of dental pulp-derived stem cells or the like is a culture supernatant of dental pulp-derived stem cells or the like of a patient with any of various diseases (viral infection and the like), the application of the cytokine storm suppressor of the invention to such a patient will be safer and cause less ethical issues.

The cytokine storm suppressor of the invention is derived from a culture supernatant of dental pulp-derived stem cells or the like and thus is used also for applications in restorative medicine. In particular, a composition containing microparticles derived from a culture supernatant of dental pulp-derived stem cells or the like is preferably used for applications in restorative medicine. Here, in regenerative medicine based on stem cell transplantation, it is known that stem cells do not play the main role in regeneration and that liquid components produced by the stem cells repair the organ with the autologous stem cells. Difficult problems of the conventional stem cell transplantation, such as malignant transformation, standardization, the administration method, preservation and the culture method, are solved, and restorative medicine is enabled by the composition using microparticles derived from a culture supernatant of dental pulp-derived stem cells or the like. Compared to stem cell transplantation, restoration using the cytokine storm suppressor of the invention does not easily cause neoplastic transformation or the like and could be considered to be safer because cell transplantation is not required. Moreover, a culture supernatant of dental pulp-derived stem cells or the like and the cytokine storm suppressor of the invention have advantages because those with uniformly standardized quality can be used. Because mass production and an efficient administration method can be selected, use with a low cost is enabled.

### <Microparticles>

In the invention, microparticles derived from a culture supernatant of dental pulp-derived stem cells or the like are used, and the microparticles contain extracellular superoxide dismutase (SOD3) in an effective amount capable of repairing a tissue or a cell damaged by a cytokine storm or a higher amount. The microparticles are derived from dental pulp-derived stem cells or the like, for example, through secretion, emergence, dispersion or the like from the dental pulp-derived stem cells or the like and are exuded or released or fall into a cell culture medium. Thus, the microparticles are contained in a culture supernatant of dental pulp-derived stem cells or the like.

When the cytokine storm suppressor of the invention is used, the microparticles derived from a culture supernatant of dental pulp-derived stem cells, adipose-derived stem cells, bone marrow-derived stem cells, umbilical cord-derived stem cells or immortalized stem cells thereof are purified from the culture supernatant. That is, the microparticles are preferably microparticles purified from the culture supernatant.

The origin of the microparticles can be identified by a known method. For example, whether the microparticles are derived from dental pulp-derived stem cells, adipose-derived stem cells, bone marrow-derived stem cells or umbilical cord-derived stem cells can be determined by the method described in J Stem Cell Res Ther (2018) 8:2. Specifically, based on the miRNA pattern of the microparticles, the origin of the microparticles can be identified.

In the invention, the microparticles contain extracellular superoxide dismutase (SOD3). Here, SOD3 is a kind of SOD. SODs are enzymes causing disproportionation of superoxide anion radical (O₂⁻) into oxygen (O₂) and hydrogen peroxide (H₂O₂). SODs are unevenly distributed in cells that metabolize oxygen and can protect the cells from free radical damage mediated by oxygen. SODs are classified into four kinds depending on the difference in the metal cofactor and the localization, and SOD3 is SOD bound to the extracellular fluid or the membrane.

### (Characteristics of Microparticles)

The microparticles are preferably of at least one kind selected from the group consisting of exosomes, microvesicles, membrane particles, membrane vesicles, ectosomes, exovesicles and microvesicles, more preferably exosomes.

The diameter of the microparticles is preferably 10 to 1000 nm, more preferably 30 to 500 nm, particularly preferably 50 to 150 nm.

The microparticles contain SOD3 in an effective amount capable of repairing a tissue or a cell damaged by a cytokine storm or a higher amount. The microparticles preferably contain 1.0 ng/mg or more of SOD3 per 1 mg of the microparticles, more preferably contain 3.0 ng/mg or more, particularly preferably contain 6.0 ng/mg or more and further particularly preferably contain 10.0 ng/mg or more. In a preferable aspect of the cytokine storm suppressor of the invention, the SOD3 activity is further improved because specific microparticles containing sufficient SOD3 in this manner are contained preferably at a high concentration.

The microparticles more preferably have a high SOD3 activity, from the viewpoint that suppression of a cytokine storm is easier.

Moreover, molecules called tetraspanins such as CD9, CD63 and CD81 are desirably on the surface of the microparticles, and the molecules may be those of CD9 alone, CD63 alone, CD81 alone or any combination of two or three thereof.

A preferable aspect in which exosomes are used as the microparticles is sometimes explained below, but the microparticles of the invention are not limited to exosomes.

The exosomes are preferably extracellular vesicles which are released from cells during fusion of plasma membrane and multivesicular bodies.

The surface of the exosomes preferably contains a lipid and a protein derived from the cell membrane of dental pulp-derived stem cells or the like.

The exosomes preferably contain, in their inside, an intracellular substance of dental pulp-derived stem cells or the like such as nucleic acids (microRNAs, messenger RNAs, DNAs and the like) and proteins.

The exosomes are known to be used for communication among cells through transportation of genetic information from a cell to another cell. The exosomes can be tracked easily and can be targeted at a specific region.

### (Microparticle Content)

The microparticle content of the cytokine storm suppressor of the invention is not particularly restricted. The cytokine storm suppressor of the invention preferably contains 0.5×10⁸ particles or more, more preferably 1.0×10⁸ particles or more, particularly preferably 2.0×10⁸ particles or more, further particularly preferably 1.0×10⁹ particles or more, still further particularly preferably 2.0×10⁹ particles or more of the microparticles.

The concentration of the microparticles contained in the cytokine storm suppressor of the invention is not particularly restricted. The cytokine storm suppressor of the invention preferably contains the microparticles at 0.01×10⁸ particles/mL or more, more preferably at 0.05×10⁸ particles/mL or more, particularly preferably at 0.1×10⁸ particles/mL or more, further particularly preferably at 1.0×10⁹ particles/mL or more, still further particularly preferably at 2.0×10⁹ particles/mL or more. In a preferable aspect of the cytokine storm suppressor of the invention, the SOD3 activity is further improved because specific microparticles containing sufficient SOD3 are contained at a high concentration in this manner.

### (Culture Supernatant of Dental Pulp-Derived Stem Cells or the Like)

The culture supernatant of dental pulp-derived stem cells or the like is not particularly restricted.

The culture supernatant of dental pulp-derived stem cells or the like preferably does not substantially contain serum. For example, the serum content of the culture supernatant of dental pulp-derived stem cells or the like is preferably 1 mass% or less, more preferably 0.1 mass% or less, particularly preferably 0.01 mass% or less.

### - Dental Pulp-Derived Stem Cells or the Like -

The dental pulp-derived stem cells, the adipose-derived stem cells, the bone marrow-derived stem cells or the umbilical cord-derived stem cells may be derived from a human or from a nonhuman animal. The nonhuman animal may be the same as the animal (species) as the subject of the administration of the cytokine storm suppressor of the invention described below and is preferably a mammal.

The dental pulp-derived stem cells used for the culture supernatant are not particularly restricted. Dental pulp stem cells from exfoliated deciduous teeth, deciduous dental pulp stem cells obtained by another method and permanent dental pulp stem cells (DPSCs) can be used. In addition to human deciduous dental pulp stem cells and human permanent dental pulp stem cells, dental pulp-derived stem cells derived from a nonhuman animal such as pig deciduous dental pulp stem cells can be used.

The dental pulp-derived stem cells (the same applies to the adipose-derived stem cells, the bone marrow-derived stem cells and the umbilical cord-derived stem cells described below) can produce, in addition to exosomes, vascular endothelial growth factors (VEGFs), hepatocyte growth factors (HGFs), insulin-like growth factors (IGFs), platelet-derived growth factors (PDGFs), transforming growth factors-beta (TGF-β)-1 and -3, TGF-α, KGF, HBEGF, SPARC, other growth factors and various cytokines such as chemokine. Many other bioactive substances can also be produced.

In the invention, the dental pulp-derived stem cells used for the culture supernatant of dental pulp-derived stem cells are particularly preferably dental pulp-derived stem cells containing many proteins, and deciduous dental pulp stem cells are preferably used. That is, in the invention, a culture supernatant of deciduous dental pulp stem cells is preferably used.

The adipose-derived stem cells used for the culture supernatant are not particularly restricted. As the adipose-derived stem cells, somatic stem cells contained in any adipose tissue can be used. In addition to human adipose-derived stem cells, adipose-derived stem cells derived from a nonhuman animal such as pig adipose stem cells can be used. As the adipose-derived stem cells, for example, adipose-derived stem cells prepared by the method described in [0023] to [0041] of WO2018/038180 can be used, and the contents of the publication are incorporated in the present description by reference. Moreover, adipose-derived stem cells prepared by the method described in [0022] and [0172] to [0187] of JP2018-531979A can be used, and the contents of the publication are incorporated in the present description by reference.

The bone marrow-derived stem cells used for the culture supernatant are not particularly restricted. In addition to human bone marrow-derived stem cells, bone marrow-derived stem cells derived from a nonhuman animal such as pig bone marrow stem cells can be used. As the bone marrow-derived stem cells, for example, bone marrow-derived stem cells prepared by the method described in [0027] to [0028] and [0048] of JP2017-132744A can be used, and the contents of the publication are incorporated in the present description by reference.

The umbilical cord-derived stem cells used for the culture supernatant are not particularly restricted. In addition to human umbilical cord-derived stem cells, umbilical cord-derived stem cells derived from a nonhuman animal such as pig umbilical cord stem cells can be used. As the umbilical cord-derived stem cells, for example, umbilical cord-derived stem cells prepared by the method described in [0023] to [0033] of JP2017-119646A can be used, and the contents of the publication are incorporated in the present description by reference.

The dental pulp-derived stem cells or the like used in the invention may be natural cells or genetically modified cells as long as the intended treatment can be achieved.

In particular, in the invention, immortalized stem cells of dental pulp-derived stem cells, adipose-derived stem cells, bone marrow-derived stem cells or umbilical cord-derived stem cells can be used. Using immortalized stem cells, which are capable of proliferating substantially infinitely, the amounts and the compositions of the biological factors contained in the culture supernatant of the stem cells can be stabilized over a long period of time. The immortalized stem cells of dental pulp-derived stem cells, adipose-derived stem cells, bone marrow-derived stem cells or umbilical cord-derived stem cells are not particularly restricted. The immortalized stem cells are preferably non-tumor immortalized stem cells. The immortalized stem cells of dental pulp-derived stem cells, adipose-derived stem cells, bone marrow-derived stem cells or umbilical cord-derived stem cells can be prepared by adding one of the following small molecule compounds (inhibitors) or any combination thereof to dental pulp-derived stem cells, adipose-derived stem cells, bone marrow-derived stem cells or umbilical cord-derived stem cells and culturing the cells.

TGFβ receptor inhibitors are not particularly limited as long as the TGFβ receptor inhibitors have the action of inhibiting the function of transforming growth factor (TGF) β receptor, and examples thereof include 2-(5-benzo[1,3]dioxol-4-yl-2-tert-butyl-1H-imidozol-4-yl)-6-methylpyridine, 3-(6-methylpyridin-2-yl)-4-(4-quinolyl)-1-phenylthiocarbamoyl-1H-pyrazole (A-83-01), 2-(5-chloro-2-fluorophenyl)pteridin-4-yl)pyridin-4-ylamine (SD-208), 3-(pyridin-2-yl)-4-(4-quinonyl)]-1H-pyrazole and 2-(3-(6-methylpyridin-2-yl)-1H-pyrazol-4-yl)-1,5-naphthyridine (all are from Merck KGaA), SB431542 (Sigma-Aldrich Co. LLC) and the like. A-83-01 is preferable.

ROCK inhibitors are not particularly limited as long as the ROCK inhibitors have the action of inhibiting the function of Rho-binding kinase. Examples of the ROCK inhibitors include GSK269962A (Axon medchem), Fasudil hydrochloride (Tocris Bioscience), Y-27632 and H-1152 (both are from FUJIFILM Wako Pure Chemical Corporation) and the like. Y-27632 is preferable.

GSK3 inhibitors are not particularly limited as long as GSK-3 (glycogen synthase kinase 3) is inhibited, and examples thereof include A 1070722, BIO and BIO-acetoxime (all are from Tocris Bioscience).

MEK inhibitors are not particularly limited as long as the MEK inhibitors have the action of inhibiting the function of MEK (MAP kinase-ERK kinase), and examples thereofincludeAZD6244, CI-1040 (PD184352), PD0325901, RDEA119 (BAY86-9766), SL327 and U0126-EtOH (all are from Selleck), and PD98059, U0124 and U0125 (all are from Cosmo Bio Co., Ltd.)

The dental pulp-derived stem cells or the like used in the invention are preferably genetically modified cells in which the microparticles highly express SOD3 from the viewpoint that the effects of the cytokine storm suppressor of the invention are enhanced. The technique for genetically modifying for high expression of SOD in the microparticles is not particularly restricted, and a known method can be used. For example, the method described in Mol Genet Genomic Med. (2019) 7:e831 can be used.

When the cytokine storm suppressor of the invention is used for regenerative medicine, due to the requirements of the Act on the Safety of Regenerative Medicine, the composition containing microparticles derived from a culture supernatant of dental pulp-derived stem cells, adipose-derived stem cells, bone marrow-derived stem cells, umbilical cord-derived stem cells or immortalized stem cells thereof (dental pulp-derived stem cells or the like) does not contain other somatic stem cells than the dental pulp-derived stem cells or the like. The cytokine storm suppressor of the invention may contain mesenchymal stem cells other than the dental pulp-derived stem cells or the like or other somatic stem cells but preferably does not contain such cells.

Examples of the somatic stem cells other than the mesenchymal stem cells include stem cells derived from the dermal system, the digestive system, the myeloid system, the nerve system and the like, but the somatic stem cells are not limited to the examples. Examples of the somatic stem cells of the dermal system include epithelial stem cells and hair follicle stem cells. Examples of the somatic stem cells of the digestive system include pancreatic (general) stem cells and hepatic stem cells. Examples of the somatic stem cells of the myeloid system (except for the mesenchymal stem cells) include hematopoietic stem cells. Examples of the somatic stem cells of the nerve system include neural stem cells and retinal stem cells.

The cytokine storm suppressor of the invention may contain stem cells other than somatic stem cells but preferably does not contain such stem cells. The stem cells other than somatic stem cells include embryonic stem cells (ES cells), induced pluripotent stem cells (iPS cells) and embryonal carcinoma cells (EC cells).

The method for preparing the culture supernatant of dental pulp-derived stem cells, adipose-derived stem cells, bone marrow-derived stem cells, umbilical cord-derived stem cells or immortalized stem cells thereof is not particularly restricted, and a conventional method can be used.

The culture supernatant of dental pulp-derived stem cells or the like is a culture solution obtained by culturing dental pulp-derived stem cells or the like and does not contain the cells themselves. For example, a culture supernatant which can be used for the invention can be obtained by culturing dental pulp-derived stem cells or the like and then separating and removing the cell components. A culture supernatant which has been appropriately subjected to various treatments (for example, centrifugation, concentration, solvent substitution, dialysis, freezing, drying, lyophilization, dilution, desalination, preservation or the like) may also be used.

The dental pulp-derived stem cells or the like for obtaining the culture supernatant of dental pulp-derived stem cells or the like can be selected by a normal method and can be selected based on the size and the morphology of the cells or as adherent cells. The dental pulp-derived stem cells can be selected from dental pulp cells collected from an exfoliated deciduous tooth or a permanent tooth as adherent cells or passaged cells thereof. The adipose-derived stem cells can be selected from adipose cells collected from adipose tissue as adherent cells or passaged cells thereof. The umbilical cord-derived stem cells can be selected from cells collected from an umbilical cord as adherent cells or passaged cells thereof. The culture supernatant of dental pulp-derived stem cells or the like used can be a culture supernatant obtained by culturing selected stem cells.

The "culture supernatant of dental pulp-derived stem cells or the like" is preferably a culture solution which does not contain the cells themselves obtained by culturing the dental pulp-derived stem cells or the like. The culture supernatant of dental pulp-derived stem cells or the like used in the invention preferably does not contain any cells (regardless of the kinds of cells) as a whole in an aspect. Due to the feature, the composition of the aspect is clearly distinguished of course from the dental pulp-derived stem cells or the like themselves and also from various compositions containing dental pulp-derived stem cells or the like. A typical example of the aspect is a composition which does not contain dental pulp-derived stem cells or the like and which is composed only of a culture supernatant of dental pulp-derived stem cells or the like.

The culture supernatant of dental pulp-derived stem cells used in the invention may contain a culture supernatant of both deciduous dental pulp-derived stem cells and adult dental pulp-derived stem cells. The culture supernatant of dental pulp-derived stem cells used in the invention preferably contains a culture supernatant of deciduous dental pulp-derived stem cells as an active ingredient, more preferably in an amount of 50 mass% or more, preferably 90 mass% or more. The culture supernatant of dental pulp-derived stem cells used in the invention is further particularly preferably a composition composed only of a culture supernatant of deciduous dental pulp-derived stem cells.

A basal medium, a basal medium supplemented with serum and the like or the like can be used for the culture solution of dental pulp-derived stem cells or the like for obtaining the culture supernatant. Examples of the basal medium which can be used include, in addition to Dulbecco's modified Eagle's medium (DMEM), Iscove's modified Dulbecco's medium (IMDM) (GIBCO and the like), Ham's F 12 medium (Sigma-Aldrich Co. LLC, GIBCO and the like), RPMI1640 medium and the like. Examples of the component that can be added to the medium include serum (fetal bovine serum, human serum, sheep serum and the like), serum replacements (knockout serum replacement (KSR) and the like), bovine serum albumin (BSA), antibiotics, various vitamins and various minerals.

In order to prepare "a culture supernatant of dental pulp-derived stem cells or the like" which does not contain serum, however, a serum-free medium should be used throughout the whole process or for the last passaging culture or the last passaging culture steps. For example, when dental pulp-derived stem cells or the like are cultured in a medium which does not contain serum (a serum-free medium), a culture supernatant of dental pulp-derived stem cells or the like which does not contain serum can be prepared. When passaging culture is conducted once or twice or more times and when the last passaging culture or the last passaging culture steps are conducted in a serum-free medium, a culture supernatant of dental pulp-derived stem cells or the like which does not contain serum can also be obtained. Moreover, a culture supernatant of dental pulp-derived stem cells or the like which does not contain serum can also be obtained by removing serum from the recovered culture supernatant using dialysis or solvent substitution or the like using a column.

For culturing the dental pulp-derived stem cells or the like for obtaining the culture supernatant, generally used conditions can be applied as they are. The method for preparing a culture supernatant of the dental pulp-derived stem cells or the like can be the same as the cell culture method described below except that the isolation and selection steps of the stem cells are appropriately adjusted to the type of the stem cells. One skilled in the art can appropriately isolate and select dental pulp-derived stem cells or the like according to the type of the dental pulp-derived stem cells or the like.

In order to produce a large amount of a specific kind of exosomes which contributes to the expression of SOD3 and/or the SOD3 activity, special conditions may be applied for culturing the dental pulp-derived stem cells or the like. The special conditions may be, for example, low-temperature conditions, low-oxygen conditions, microgravity conditions, conditions of coculturing with any stimulant or the like.

### - Other Components Contained in Culture Supernatant of Dental Pulp-Derived Stem Cells or the Like -

The culture supernatant of dental pulp-derived stem cells or the like used for preparing exosomes in the invention may contain other components in addition to the culture supernatant of dental pulp-derived stem cells or the like but preferably does not substantially contain any other components.

The culture supernatant of dental pulp-derived stem cells or the like may be stored after various additives used for preparing exosomes is added to the culture supernatant.

### (Purification of Microparticles)

Microparticles can be purified from the culture supernatant of dental pulp-derived stem cells or the like.

The purification of the microparticles is preferably separation of a fraction containing the microparticles from the culture supernatant of dental pulp-derived stem cells or the like, more preferably isolation of the microparticles.

The microparticles can be isolated by separation from non-associated components based on the characteristics of the microparticles. For example, the microparticles can be isolated based on the molecular weight, the size, the morphology, the composition or the biological activity.

In the invention, the microparticles can be purified by collecting a specific fraction containing a large amount of the microparticles (for example, precipitates) obtained by centrifuging the culture supernatant of dental pulp-derived stem cells or the like. Unnecessary components (insoluble components) in a fraction other than the predetermined fraction may be removed. The solvent, the dispersion medium and the unnecessary components do not have to be removed completely from the cytokine storm suppressor of the invention. An example of the centrifugation conditions is 100 to 20000 g for 1 to 30 minutes.

In the invention, the microparticles can be purified by subjecting the culture supernatant of dental pulp-derived stem cells or the like or a centrifuged product thereof to filtration. Unnecessary components can be removed through the filtration. Moreover, when a filter membrane having an appropriate pore size is used, the removal of the unnecessary components and sterilization can be conducted simultaneously. The material, the pore size and the like of the filter membrane used for the filtration are not particularly limited. The filtration can be achieved by a known method using a filter membrane having an appropriate molecular weight or an appropriate size cut-off. From the viewpoint that exosomes can be easily fractionated, the pore size of the filter membrane is preferably 10 to 1000 nm, more preferably 30 to 500 nm, particularly preferably 50 to 150 nm.

In the invention, the culture supernatant of dental pulp-derived stem cells or the like, a centrifuged product thereof or a filtered product thereof can be separated further using separation means such as RAM chromatography. For example, high-performance liquid chromatography (HPLC) using any of various columns can be used. The column which can be used is a size exclusion column or a binding column.

In order to track the microparticles (or the activity thereof) in the fractions at each treatment stage, one or more characteristics or the biological activity of the microparticles can be used. For example, in order to track the microparticles, light scattering, refractive index, dynamic light scattering or UV-VIS detector can be used. Alternatively, in order to track the activity in each fraction, the expression level of SOD3, the SOD3 activity or the like can be used.

As the method for purifying the microparticles, the method described in [0034] to [0064] of JP2019-524824A may be used, and the contents of the publication are incorporated in the present description by reference.

### <Other Components>

The cytokine storm suppressor of the invention may contain other components in addition to the microparticles depending on the species of the animal as the subject of the administration or the purpose as long as the effects of the invention are not impaired. Examples of the other components include nutritional components, antibiotics, cytokines, protectants, carriers, excipients, disintegrating agents, buffers, emulsifiers, suspending agents, soothing agents, stabilizers, preservatives, antiseptics and the like.

Examples of the nutritional components include fatty acids and vitamins.

Examples of the antibiotics include penicillin, streptomycin and gentamicin.

The carriers may be materials known as pharmaceutically acceptable carriers.

The cytokine storm suppressor of the invention may be the microparticles themselves or a pharmaceutical composition further containing a pharmaceutically acceptable carrier or excipient or the like. The purpose of the pharmaceutical composition is to promote administration of the microparticles to the subject of the administration.

The pharmaceutically acceptable carrier is preferably a carrier (including a diluent) which does not cause significant irritation in the subject of the administration and which does not inhibit the biological activity and the characteristics of the administered composition. Examples of the carrier include propylene glycol, (physiological) saline, an emulsion, a buffer, a medium such as DMEM and RPMI, and a low-temperature preservation medium containing a component for removing free radicals.

The cytokine storm suppressor of the invention may contain the active ingredient of a conventionally known cytokine storm suppressor and/or an immunosuppressive agent. For example, the cytokine storm suppressor of the invention preferably contains an active ingredient having a different mechanism from that of the invention, such as an anti-IL-6 antibody and a steroid, from the viewpoint that the effects are enhanced, balanced and/or complemented. The cytokine storm suppressor of the invention more preferably contains at least one of an anti-IL-6 antibody and a steroid and particularly preferably contains an anti-IL-6 antibody. When the cytokine storm suppressor of the invention contains an active ingredient having a different mechanism from that of the invention, such as an anti-IL-6 antibody and a steroid, the amount or the concentration of such an active ingredient may be the same as or different from that of a known method. One skilled in the art can appropriately change the amount or the concentration depending on the use, the subject of the administration and the like.

The cytokine storm suppressor of the invention preferably does not contain specific substances.

For example, the cytokine storm suppressor of the invention preferably does not contain dental pulp-derived stem cells or the like.

Moreover, the cytokine storm suppressor of the invention preferably does not contain MCP-1. In this regard, however, cytokines other than MCP-1 may be contained. Examples of the other cytokines include those described in [0014] to [0020] of JP2018-023343A.

The cytokine storm suppressor of the invention preferably does not contain Siglec-9. In this regard, however, sialic acid-binding immunoglobulin-like lectins other than Siglec-9 may be contained.

The cytokine storm suppressor of the invention preferably does not substantially contain serum (fetal bovine serum, human serum, sheep serum or the like). Moreover, the cytokine storm suppressor of the invention preferably does not substantially contain the conventional serum replacements such as knockout serum replacement (KSR).

The amounts (solid amounts) of the other components in the cytokine storm suppressor of the invention are each preferably 1 mass% or less, more preferably 0.1 mass% or less, particularly preferably 0.01 mass% or less.

### <Production Method of Cytokine Storm Suppressor>

The method for producing the cytokine storm suppressor of the invention is not particularly restricted.

The cytokine storm suppressor of the invention may be prepared by preparing a culture supernatant of dental pulp-derived stem cells or the like by the above method and subsequently purifying microparticles from the culture supernatant of dental pulp-derived stem cells or the like. Alternatively, the cytokine storm suppressor of the invention may be prepared by purifying microparticles from a commercially purchased culture supernatant of dental pulp-derived stem cells or the like. The cytokine storm suppressor of the invention may also be prepared by receiving a composition containing a culture supernatant of dental pulp-derived stem cells or the like which has been disposed of (or by appropriately purifying such a composition) and purifying microparticles from the composition.

The final form of the cytokine storm suppressor of the invention is not particularly restricted. For example, the cytokine storm suppressor of the invention may be in a form in which the microparticles are packed in a container with a solvent or a dispersion medium, a form in which the microparticles are formed into gel with gel and packed in a container, a form in which the microparticles are solidified by freezing and/or drying and formulated or packed in a container or another form. Examples of the container include a tube, a centrifuge tube or a bag which is suitable for cryopreservation. The freezing temperature can be, for example, -20°C to -196°C.

### [Use Method of Cytokine Storm Suppressor]

The method for using a cytokine storm suppressor of the invention includes administering the cytokine storm suppressor of the invention to a human or a nonhuman animal to decrease the cellular or blood cytokine amount of the human or the nonhuman animal and repair a tissue damaged by a cytokine storm through suppression of the cytokine storm.

The step of administering the cytokine storm suppressor of the invention to a human or a nonhuman animal is not particularly restricted.

The administration method can be spraying or inhalation to the oral cavity, the nasal cavity or the respiratory tract, infusion, local administration, nasal drops or the like and is preferably little invasive. The local administration method is preferably an injection. Another preferable method is electroporation, in which voltage (electric pulses) is applied to the skin surface to create temporal fine holes in the cell membrane, allowing the active ingredient to penetrate to the dermis layer, which cannot be reached by normal care. The local administration may be intravenous administration, intraarterial administration, intraportal administration, intradermal administration, subcutaneous administration, intramuscular administration, intraperitoneal administration or the like and is more preferably intraarterial administration or intravenous administration, more preferably intravenous administration.

The cytokine storm suppressor of the invention is not limited to application to a lung tissue but can be applied to the tissues of the whole body (all organs, vessels and the like). Accordingly, the administration method is more preferably intraarterial administration or intravenous administration, rather than spraying or inhalation to the oral cavity, the nasal cavity or the respiratory tract, from the viewpoint that the application to the tissues of the whole body is easy, and intravenous administration is particularly preferable. The cytokine storm suppressor of the invention which has been administered to an animal circulates in the body of the animal and may reach a certain tissue.

The number of administrations and the intervals of administration are not particularly restricted. The number of administrations can be once to twice or more and is preferably once to five times, more preferably once or twice, particularly preferably twice. The intervals of administration are preferably 1 to 24 hours, more preferably within 12 hours. However, the number and the intervals can be appropriately adjusted in accordance with the symptom of the subject of the administration.

The dosage is not particularly restricted because the microparticles contain extracellular superoxide dismutase (SOD3) in an effective amount capable of repairing a tissue or a cell damaged by a cytokine storm or a higher amount. In a mouse model, the dosage is preferably 1 to 50 µg, more preferably 3 to 25 µg, further particularly preferably 5 to 15 µg per one mouse (about 25 g). In the case of administration to another animal, the dosage is preferably 0.04 to 2 mg/kg (weight/body weight), more preferably 0.1 to 1 mg/kg, particularly preferably 0.2 to 0.6 mg/kg. The dosage can be appropriately adjusted depending on the symptom of the subject of the administration.

The timing for starting the administration is not particularly restricted. The timing for starting the administration to a human or a nonhuman animal with a cytokine storm may be before damage of a tissue is caused by the cytokine storm or after damage of a tissue is caused by the cytokine storm. In the invention, administration to a human or a nonhuman animal with a cytokine storm before damage of a tissue is caused by the cytokine storm is preferable to further increase the survival rate. That is, the cytokine storm suppressor of the invention and the use method thereof are preferably used for administration to a human or a nonhuman animal with a cytokine storm before damage of a tissue is caused by the cytokine storm to suppress the damage of a tissue by the cytokine storm through suppression of the cytokine storm or used for repairing a tissue to be damaged by the cytokine storm. The cytokine storm suppressor of the invention and the use method thereof are more preferably used for administration to a human or a nonhuman animal with a cytokine storm before damage of a tissue is caused by the cytokine storm to suppress the damage of a tissue by the cytokine storm through suppression of the cytokine storm.

Moreover, the cytokine storm suppressor of the invention and the use method thereof are also preferably used for administration to a human or a nonhuman animal with damage of a tissue caused by a cytokine storm to repair the tissue damaged by the cytokine storm through suppression of the cytokine storm.

Regarding the timing for starting the administration, the survival rate was shown to increase with the administration six hours and 18 hours after the LPS administration in Fig. 6, namely, administration to a human or a nonhuman animal with damage of a tissue caused by a cytokine storm. Compared to the case in Fig. 6, the survival rate was shown to further increase with the administration 0 hour (the moment the cytokine storm was caused) and 12 hours after the LPS administration in Fig. 7, namely administration to a human or a nonhuman animal with a cytokine storm before damage of a tissue is caused by the cytokine storm. In this regard, the state which has progressed to viral infection of a human or a nonhuman animal, virus proliferation and the occurrence of a cytokine storm by immune cells and which is before damage of a tissue is caused by the cytokine storm is the state of 0 hour after the LPS administration.

To increase the survival rate, specific timing for starting the administration is preferably within six hours after the occurrence of the cytokine storm, more preferably within three hours, particularly preferably within one hour, further particularly preferably the same as the timing at which the cytokine storm is (believed to be) caused.

The animal (species) as the subject to which the cytokine storm suppressor of the invention is administered is not particularly restricted. The animal as the subject to which the cytokine storm suppressor of the invention is administered is preferably a mammal, a bird (chicken, quail, duck or the like) or fish (salmon, trout, tuna, bonito or the like). The mammal may be a human or a nonhuman mammal but is particularly preferably a human. The nonhuman mammal is more preferably a cow, a pig, a horse, a goat, a sheep, a monkey, a dog, a cat, a mouse, a rat, a guinea pig or a hamster.

The cytokine storm suppressor of the invention may be used in combination with a conventionally known cytokine storm suppressor and/or an immunosuppressive agent. For example, the cytokine storm suppressor of the invention is preferably used in combination with a drug having a different mechanism from that of the invention, such as an anti-IL-6 antibody and a steroid, from the viewpoint of the effects are enhanced, balanced and/or complemented. In the method for using a cytokine storm suppressor of the invention, the cytokine storm suppressor of the invention is more preferably used in combination with at least one of an anti-IL-6 antibody and a steroid and is particularly preferably used in combination with an anti-IL-6 antibody. When the cytokine storm suppressor of the invention and a drug having a different mechanism from that of the invention, such as an anti-IL-6 antibody and a steroid, are used in combination, the dosage, the number of administrations and the timing of administration of such a drug may be the same as or different from those of a known method. Moreover, when the cytokine storm suppressor of the invention and a drug having a different mechanism from that of the invention, such as an anti-IL-6 antibody and a steroid, are used in combination, the numbers of administrations and the timings of administration of the two may be the same or different. One skilled in the art can appropriately change the numbers and the timings depending on the use, the subject of the administration and the like.

An anti-IL-6 antibody has a mechanism of inhibiting the functions of IL-6 produced by marginal zone B cells, which is a factor that deteriorates sepsis (cytokine storm) (Nature communications (2016) 7:11498). The paper shows that, although representative inflammatory cytokines, IL-6 and TNFα, are produced in large amounts from macrophages but are produced only in small amounts from marginal zone B cells one hour after the LPS administration, marginal zone B cells produce more IL-6 than macrophages four hours after the LPS administration.

A steroid is known to have a mechanism of binding to glucocorticoids (GRs) in the cytoplasm to form active GRs, regulating the gene expression of nuclear DNA or a transcription-regulating factor and suppressing cytokine synthesis (IL-1, IL-2, IL-5, IL-6, IL-8 and IFNy), thereby exhibiting anti-inflammatory action and immunosuppressive action.

### [Screening Method]

The screening method of the invention is a screening method for selecting a factor or a combination of factors which is effective for tissue repair for repairing a tissue damaged by a cytokine storm through suppression of the cytokine storm and has a step of supplying one, two or more components contained in a culture supernatant obtained by culturing dental pulp-derived stem cells, adipose-derived stem cells, bone marrow-derived stem cells, umbilical cord-derived stem cells or immortalized stem cells thereof to an evaluation system related to extracellular superoxide dismutase (SOD3) and evaluating a SOD3-related feature.

According to the screening method of the invention, components contained in a culture supernatant of dental pulp-derived stem cells or the like can be screened to identify a component which is effective for tissue repair of repairing a tissue damaged by a cytokine storm through suppression of the cytokine storm, by supplying the components to an evaluation system related to SOD3 and evaluating a SOD3-related feature. In the screening method of the invention, for example, a component having a SOD3 protein amount of preferably 1.0 ng/mg or more after purification is preferably identified and obtained, as a result of evaluation of a SOD3-related feature of the components. Moreover, for example, a component having a SOD3 activity of preferably 0.3 unit/µg or more after purification is preferably identified and obtained.

Moreover, according to the screening method of the invention, a cytokine storm suppressor or a prophylactic or therapeutic composition containing a component identified by the screening as an active ingredient can be obtained. Regarding the cytokine storm suppressor or the prophylactic or therapeutic composition obtained by the screening, an effective cytokine storm suppressor or an effective prophylactic or therapeutic composition can be obtained by combining specific components which are commercially available, purified and/or obtained in another way without deriving the components from a culture supernatant of dental pulp-derived stem cells or the like. That is, the invention also relates to a cytokine storm suppressor which contains a component obtained by evaluation of the SOD3-related feature by the screening method of the invention and which repairs a tissue damaged by a cytokine storm through suppression of the cytokine storm, and the component is derived from a culture supernatant of dental pulp-derived stem cells, adipose-derived stem cells, bone marrow-derived stem cells, umbilical cord-derived stem cells or immortalized stem cells thereof, contains extracellular superoxide dismutase (SOD3) in an effective amount capable of repairing a tissue or a cell damaged by a cytokine storm or a higher amount and satisfies at least one of the feature that 1.0 ng/mg or more of SOD3 is contained per 1 mg of the component and the feature that the SOD3 activity is 0.3 unit/µg or more.

More preferable ranges of the SOD3 protein amount and the SOD activity of the component are similar to the more preferable ranges of the SOD3 protein amount and the SOD activity of the cytokine storm suppressor using microparticles derived from a culture supernatant of dental pulp-derived stem cells, adipose-derived stem cells, bone marrow-derived stem cells, umbilical cord-derived stem cells or immortalized stem cells thereof.

As the evaluation system related to SOD3 used in the screening method of the invention, a known evaluation system can be used. For example, the method for measuring SOD3 and the method for measuring the SOD3 activity described in the present description can be applied to model mice for cytokine storm-related diseases, and an evaluation system using related cells can be appropriately selected and used. One skilled in the art can appropriately select an evaluation system of such a type and can refer to Examples of the present description.

The component obtained by evaluation of the SOD3-related feature by the screening method of the invention may be microparticles (preferably exosomes) derived from a culture supernatant of dental pulp-derived stem cells or the like, as described above.

In the screening method of the invention, a SOD3-related feature of other components derived from a culture supernatant of dental pulp-derived stem cells or the like can be evaluated, and the component can be obtained. As described above, a culture supernatant of dental pulp-derived stem cells, adipose-derived stem cells, bone marrow-derived stem cells, umbilical cord-derived stem cells or immortalized stem cells thereof contains, in addition to microparticles such as exosomes, VEGF, HGF, IGF, PDGF, TGF-β-1 and -3, TGF-α, KGF, HBEGF, SPARC, other growth factors, various cytokines such as chemokine and many other bioactive substances.

### Examples

The characteristics of the invention are explained further specifically referring to Examples and Comparative Examples or Reference Examples below. The materials, the amounts, the proportions, the contents of treatments, the procedures of treatments and the like shown in Examples below can be appropriately changed as long as such changes do not go beyond the intention of the invention. Accordingly, the scope of the invention should not be construed as being limited by the specific examples shown below.

### [Example 1]

### <Preparation of Culture Supernatant of Dental Pulp-Derived Stem Cells>

A culture supernatant of human deciduous dental pulp stem cells was prepared according to the method described in Example 6 of JP6296622B except that DMEM medium was used instead of the DMEM/HamF 12 mixture medium. For the primary culture, fetal bovine serum (FBS) was added to the culture. In the passaging culture, the supernatant was taken from the passaged culture solution which was cultured using the primary culture solution in such a manner that FBS would not be contained, and a culture supernatant of deciduous dental pulp stem cells was thus prepared. Here, DMEM is Dulbecco's modified Eagle's medium, and F 12 is Ham's F 12 medium.

### <Preparation of Exosomes from Culture Supernatant of Dental Pulp-Derived Stem Cells>

Exosomes of the dental pulp-derived stem cells were purified from the obtained culture supernatant of deciduous dental pulp stem cells by the following method.

After filtering the culture supernatant of deciduous dental pulp stem cells (100 mL) with a filter having a pore size of 0.22 micrometers, the solution was centrifuged for 60 minutes at 4°C at 100000× g. The supernatant was decanted, and the exosome-concentrated pellets were re-suspended in phosphate-buffered saline (PBS). The re-suspended sample was centrifuged for 60 minutes at 100000× g. The pellets were again recovered from the bottom of the centrifuge tube as the concentrated sample (about 100 µl). The protein concentration was determined using micro BSA protein assay kit (Pierce, Rockford, IL). The composition containing exosomes (concentrated solution) was stored at -80°C.

The obtained composition containing exosomes (dental pulp-derived stem cell EVs) was used as the cytokine storm suppressor of Example 1.

### [Example 2] Preparation of Adipose-Derived Stem Cell EVs (Exosomes)

A culture supernatant of adipose-derived stem cells was prepared in the same manner as in Example 1 except for using adipose-derived stem cells. A composition containing exosomes was prepared in the same manner as in Example 1 from the obtained culture supernatant of adipose-derived stem cells. The obtained composition containing exosomes (adipose-derived stem cell EVs) was used as the cytokine storm suppressor of Example 2.

### [Example 3] Preparation of Bone Marrow-Derived Stem Cell EVs (Exosomes)

A culture supernatant of bone marrow-derived stem cells was prepared in the same manner as in Example 1 except for using bone marrow-derived stem cells. A composition containing exosomes was prepared in the same manner as in Example 1 from the obtained culture supernatant of bone marrow-derived stem cells. The obtained composition containing exosomes (bone marrow-derived stem cell EVs) was used as the cytokine storm suppressor of Example 3.

### [Example 4] Preparation of Umbilical Cord-Derived Stem Cell EVs (Exosomes)

A culture supernatant of umbilical cord-derived stem cells was prepared in the same manner as in Example 1 except for using umbilical cord-derived stem cells. A composition containing exosomes was prepared in the same manner as in Example 1 from the obtained culture supernatant of umbilical cord-derived stem cells. The obtained composition containing exosomes (umbilical cord-derived stem cell EVs) was used as the cytokine storm suppressor of Example 4.

### [Evaluation]

### <Characteristics of Exosomes>

The average particle sizes of the microparticles contained in the cytokine storm suppressors of the Examples were 50 to 150 nm.

The cytokine storm suppressors of the Examples were exosome solutions having a high concentration of 1.0×10⁹ particles/ml or more, and in particular, the cytokine storm suppressor of Example 1 was an exosome solution having a high concentration of 2.0×10⁹ particles/ml.

Moreover, the components of the obtained cytokine storm suppressors of the Examples were analyzed by a known method. As a result, it was found that the cytokine storm suppressors of the Examples did not contain any stem cells such as dental pulp-derived stem cells, did not contain MCP-1 and did not contain Siglec-9. It was thus found that an active ingredient which is different from MCP-1 and Siglec-9, which are active ingredients of a culture supernatant of mesenchymal stem cells, and from analogs thereof is the active ingredient of the cytokine storm suppressors of the Examples.

### <Measurement of SOD3>

Using SOD3 (Human) ELISA Kit (manufactured by A-Frontier Co., Ltd.(LSR), model number LFEK0107), the SOD3 protein amounts of the dental pulp-derived stem cell EVs (the cytokine storm suppressor of Example 1), the adipose-derived stem cell EVs (the cytokine storm suppressor of Example 2), the umbilical cord-derived stem cell EVs (the cytokine storm suppressor of Example 3), the bone marrow-derived stem cell EVs (the cytokine storm suppressor of Example 4) and DMEM medium used as a control were measured by the sandwich ELISA (N=2). The obtained results are shown in Fig. 1.

From Fig. 1, it was found that the exosomes contained in the cytokine storm suppressors of the Examples contained a large amount of SOD3. In particular, the dental pulp-derived stem cell EVs (Example 1) were found to have a higher SOD3 protein amount than the adipose-derived stem cell EVs (Example 2), the bone marrow-derived stem cell EVs (Example 3) or the umbilical cord-derived stem cell EVs (Example 4). From the results, the cytokine storm suppressors of the invention were found to contain SOD3 at a high concentration and be thus able to regulate the expression of cytokines. Moreover, as the SOD3 protein amount is higher, the regulation of the expression of cytokines becomes easier, and the amounts of cytokines in a cytokine storm state can be reduced more easily.

### <Measurement of SOD3 Activity>

Using xanthine oxidase method using Stressxpress Superoxide Dismutase Kit (manufactured by StressMarq Biosciences INC), the SOD3 (Human) activities of the dental pulp-derived stem cell EVs (the cytokine storm suppressor of Example 1), the adipose-derived stem cell EVs (the cytokine storm suppressor of Example 2), the umbilical cord-derived stem cell EVs (the cytokine storm suppressor of Example 3) and the bone marrow-derived stem cell EVs (the cytokine storm suppressor of Example 4) were measured.

For the measurement, the exosome concentrations and the amounts of the cytokine storm suppressors of the Examples were made the same to make the exosome amounts the same. The obtained results are shown in Table 1 below.

**[Table 1]**

| | Microparticles | SOD3 Activity (umt/µg) |
|---|---|---|
| Example 1 | Dental pulp-derived stem cell EVs | 3.05 |
| Example 2 | Adipose-derived stem cell EVs | 4.39 |
| Example 3 | Bone marrow-derived stem cell EVs | 0.48 |
| Example 4 | Umbilical cord-derived stem cell EVs | 1.27 |

From Table 1 above, it was found that the cytokine storm suppressors of the Examples had a high SOD3 activity. In particular, the dental pulp-derived stem cell EVs (Example 1) were found to have a higher SOD3 activity than the adipose-derived stem cell EVs (Example 2), the bone marrow-derived stem cell EVs (Example 3) or the umbilical cord-derived stem cell EVs (Example 4). The measurement of the SOD3 activity is more accurate than the measurement of the SOD3 protein amount as the method for observing the easiness of regulating the cytokine amount. As the SOD3 activity is higher, the regulation of the expression of cytokines becomes easier, and the amounts of types of cytokines in a cytokine storm state can be reduced more easily.

*<In Vitro* Observation of Cytokine Storm Suppression>

A cytokine storm was induced using human peripheral-blood mononuclear cells (PBMCs), and the cytokine storm suppression effects of the cytokine storm suppressors of the Examples containing exosomes were observed *in vitro* by the following method.

Human PMBCs were cultured and stimulated with a cytokine storm inducer, Concanavalin A (Con-A, manufactured by FUJIFILM Wako Pure Chemical Corporation), and thus a cytokine storm was induced.

The system was subjected to treatment of adding one kind of the cytokine storm suppressors of the Examples and physiological saline as a control, and the quantity of cytokines contained in the culture supernatants of human PMBCs was determined with a cytokine measurement ELISA kit (manufactured by R&D Systems, Inc., product name Quantikine ELISA kit). The quantified cytokines are IL-2, IL-4, IL-6, IL-10, IL-17, IFNγ and TNFα.

The addition of the cytokine storm suppressors of Examples 1 to 3 containing exosomes was treated to achieve 100 particles per PMBC. The cytokine amounts were measured 48 hours after the treatment with the cytokine storm suppressors of the Examples containing exosomes (N=6).

The obtained results are shown in Table 2 below.

**[Table 2]**

| | Control | Example 1 | Example 2 | Example 3 |
|---|---|---|---|---|
| Microparticles | None (physiological saline) | Dental pulp-derived stem cell EVs | Adipose-derived stem cell EVs | Bone marrow-derived stem cell EVs |
| IL-2 (pg/ml) | 28.9±6.3 | 11.5±3.1 | 20.0±11.1 | 12.6±7.2 |
| IL-4 (pg/ml) | 19.9±12.0 | 5.7±4.1 | 6.6±3.3 | 5.5±2.8 |
| IL-6 (pg/ml) | 112.0±23.1 | 10.6±5.5 | 19.9±4.3 | 29.3±10.1 |
| IL-10 (pg/ml) | 125.2±33.9 | 100.1±12.5 | 96.6±12.9 | 111.6±20.2 |
| IL-17 (pg/ml) | 141.3±25.7 | 11.3±2.0 | 49.1±12.3 | 19.9±7.4 |
| IFNy (pg/ml) | 3045.5±12.2 | 92.9±4.3 | 233±22.3 | 198±39.0 |
| TNFα (pg/ml) | 1022±29.4 | 151.8±6.6 | 241.6±49.1 | 475.3±62.1 |

| | | | | |
|---|---|---|---|---|
| (N=6) | | | | |

From Table 2 above, it was found that the cytokine storm suppressors of the Examples could significantly suppress the cytokine amounts when the cytokine storm suppressors were administered to cells with cytokine amounts increased by an induced cytokine storm compared to the control to which physiological saline was administered. In particular, the dental pulp-derived stem cell EVs (Example 1) were found to be able to significantly suppress the cytokine amounts compared to the adipose-derived stem cell EVs (Example 2) and the bone marrow-derived stem cell EVs (Example 3).

Although the umbilical cord-derived stem cell EVs of Example 4 were not used, because the SOD3 protein amount and the SOD3 activity were between those of the adipose-derived stem cell EVs of Example 2 and the bone marrow-derived stem cell EVs of Example 3, the degree of regulation of the cytokine amounts is estimated to be in the middle of those of Examples 2 and 3.

### <In Vivo Observation of Cytokine Storm Suppression>

A cytokine storm was induced in a mouse model, and the cytokine storm suppression effects of the cytokine storm suppressors of the Examples containing exosomes were observed *in vivo* by the following method.

Six 8-week-old C57BL/6J mice were used per group.

Lipopolysaccharide (LPS), which is a cytokine storm inducer, was intraperitoneally administered to the mice at 20 mg/kg body weight.

Immediately after the lipopolysaccharide administration, one kind of the cytokine storm suppressors of the Examples (purified by ultracentrifugation) and physiological saline as a control, in the same amount of 10 µg, was administered to the mice from the tail vein.

The whole blood was taken after 24 hours, and the serum of the mice with the cytokine storm was fractionated. The quantity of cytokines contained in the serum of the mice with the cytokine storm was determined with Immune Monitoring 48-Plex Mouse ProcartaPlex (registered trademark) Panel (manufactured by Invitrogen). In addition, the whole blood was taken from normal mice before the lipopolysaccharide administration, and the quantity of cytokines contained in the serum was determined in the same manner. The quantified cytokines are IL-2, IL-4, IL-6, IL-10, IL-17, IFNγ and TNFα.

The obtained results are shown in Table 3 below.

**[Table 3]**

| | Normal Mouse | Control | Example 1 | Example 2 | Example 3 |
|---|---|---|---|---|---|
| Microparticles | None (without cytokine storm induction) | None (physiological saline) | Dental pulp-derived stem cell EVs | Adipose-derived stem cell EVs | Bone marrow-derived stem cell EVs |
| IL-2 (pg/ml) | ND | 3420±44 | 158±21 | 1004±52 | 889±29 |
| IL-4 (pg/ml) | ND | 2005±27 | 509±20 | 401±35 | 1005±27 |
| IL-6 (pg/ml) | 31±2 | 1502±46 | 607±18 | 699±31 | 902±43 |
| IL-10 (pg/ml) | 19±9 | 4438±97 | 1221±45 | 2359±275 | 1055±74 |
| IL-17 (pg/ml) | ND | 2543±47 | 1022±36 | 1977±52 | 1354±25 |
| IFNy (pg/ml) | ND | 986±21 | 40±3 | 55±8 | 167±24 |
| TNFα (pg/ml) | 10±4 | 202±16 | 14±2 | 46±12 | 15±2 |

| | | | | | |
|---|---|---|---|---|---|
| ND: Not Detected (N=6) | | | | | |

From Table 3 above, it was found that the cytokine storm suppressors of the Examples could significantly suppress the cytokine amounts when the cytokine storm suppressors were administered to an animal (mouse) with cytokine amounts increased by an induced cytokine storm compared to the control to which physiological saline was administered. In particular, the dental pulp-derived stem cell EVs (Example 1) were found to be able to significantly suppress the cytokine amounts compared to the adipose-derived stem cell EVs (Example 2) and the bone marrow-derived stem cell EVs (Example 3).

Although the umbilical cord-derived stem cell EVs of Example 4 were not used, because the SOD3 protein amount and the SOD3 activity were between those of the adipose-derived stem cell EVs of Example 2 and the bone marrow-derived stem cell EVs of Example 3, the degree of regulation of the cytokine amounts is estimated that is tends to be in the middle of those of Examples 2 and 3.

### <Evaluation of Survival Rate in Mouse Model Lethally Damaged by LPS Administration (1)>

### (1) Observation of Effects of One Administration

A cytokine storm was induced in a mouse model, and the survival rate-improving effects of the cytokine storm suppressors of the Examples containing exosomes were observed *in vivo* by the following method.

Ten Slc:ICR mice were used per group.

Lipopolysaccharide (LPS), which is a cytokine storm inducer, was intraperitoneally administered to the mice at 30 mg/kg body weight. The conditions are severe conditions under which all the mice die within 96 hours.

Six hours after the LPS administration, one kind of the cytokine storm suppressors of the Examples (purified by ultracentrifugation) and physiological saline as a control, in the same amount of 10 µg per mouse, was administered to the mice from the tail vein.

The survival rates of the mice of the groups were observed every 12 hours from 12 hours after the LPS administration. The obtained results are shown in Fig. 2.

From Fig. 2, it was found that the cytokine storm suppressors of the Examples could significantly improve the survival rate when the cytokine storm suppressors were administered to an animal (mouse) with an induced cytokine storm compared to the control to which physiological saline was administered. In particular, the dental pulp-derived stem cell EVs (Example 1) were found to be able to significantly improve the survival rate compared to the adipose-derived stem cell EVs (Example 2) and the bone marrow-derived stem cell EVs (Example 3).

The improvement of the survival rate suggests that the cytokine storm suppressors of the Examples may have also repaired a tissue damaged by the cytokine storm.

### <Evaluation of Survival Rate in Mouse Model Lethally Damaged by LPS Administration (2)>

### (2) Observation of Effects of Two Administrations

A cytokine storm was induced in a mouse model, and the survival rate-improving effects of the cytokine storm suppressors of the Examples containing exosomes were observed *in vivo* by the following method.

Ten Slc:ICR mice were used per group.

Lipopolysaccharide (LPS), which is a cytokine storm inducer, was intraperitoneally administered to the mice at 30 mg/kg body weight.

Six hours after the LPS administration, one kind of the cytokine storm suppressors of the Examples (purified by ultracentrifugation) and physiological saline as a control, in the same amount of 10 µg per mouse, was administered to the mice from the tail vein as the first administration.

Eighteen hours after the LPS administration, one kind of the cytokine storm suppressors of the Examples and physiological saline as a control, in the same amount of 10 µg per mouse, was administered to the mice from the tail vein as the second administration.

The survival rates of the mice of the groups were observed every 12 hours from 12 hours after the LPS administration. The obtained results are shown in Fig. 3.

From Fig. 3, it was found that the cytokine storm suppressors of the Examples could significantly improve the survival rate when the cytokine storm suppressors were administered to an animal (mouse) with an induced cytokine storm compared to the control to which physiological saline was administered. In particular, the dental pulp-derived stem cell EVs (Example 1) were found to be able to significantly improve the survival rate compared to the adipose-derived stem cell EVs (Example 2) and the bone marrow-derived stem cell EVs (Example 3).

The improvement of the survival rate suggests that the cytokine storm suppressors of the Examples may have also repaired a tissue damaged by the cytokine storm.

Furthermore, from the comparison between the results in Fig. 2 and the results in Fig. 3, it was found that the survival rate could be significantly improved through the second administration of the cytokine storm suppressors of the Examples compared to one administration.

Here, Nature communications (2016) 7:11498 describes that, when an anti-IL-6 antibody was administered to C57BL/6 mice to which 600 µg per mouse of LPS was administered, the survival rate was 75% under the conditions where all the mice to which a control antibody was administered died 96 hours after the LPS administration. Although there are differences in the mice and the experimental system, from the descriptions of the paper and the comparison of the results in Fig. 2 and Fig. 3, the cytokine storm suppressors of the Examples (especially, the cytokine storm suppressor of Example 1) were found to have an almost equivalent effect to that of the anti-IL-6 antibody in the evaluation of the survival rate.

### <Evaluation of Survival Rate in Mouse Model Lethally Damaged by LPS Administration (3)>

### (3) Comparison of Purified Microparticles and Culture Supernatant

A cytokine storm was induced in a mouse model, and the survival rate-improving effects of the cytokine storm suppressor of Example 1 containing dental pulp-derived stem cell exosomes, compared to those of the direct use of a culture supernatant of dental pulp-derived stem cells, were observed *in vivo* by the following method.

Five Slc:ICR mice were used per group.

Lipopolysaccharide (LPS), which is a cytokine storm inducer, was intraperitoneally administered to the mice at 30 mg/kg body weight.

Six hours after the LPS administration, 10 µg per mouse of the cytokine storm suppressor of Example 1 (purified by ultracentrifugation), 0.5 ml per mouse of a culture supernatant of dental pulp-derived stem cells and 10 µg per mouse of physiological saline as a control were administered to the mice from the tail vein as the first administration. Here, 0.5 ml of the culture supernatant of dental pulp-derived stem cells used contains exosomes in an amount corresponding to 10 µg of the cytokine storm suppressor of Example 1.

Eighteen hours after the LPS administration, the cytokine storm suppressor of Example 1, the culture supernatant of dental pulp-derived stem cells and physiological saline as a control, in the same amounts as those of the first administration, were administered to the mice from the tail vein as the second administration.

The survival rates of the mice of the groups were observed every 12 hours from 12 hours after the LPS administration. The obtained results are shown in Fig. 4.

From Fig. 4, the cytokine storm suppressor of Example 1 containing dental pulp-derived stem cell exosomes was found to be able to significantly improve the survival rate when the cytokine storm suppressor was administered to an animal (mouse) with an induced cytokine storm compared to the direct use of the culture supernatant of dental pulp-derived stem cells and the control to which physiological saline was administered.

The improvement of the survival rate of the cytokine storm suppressor of Example 1 containing dental pulp-derived stem cell exosomes suggests that a tissue damaged by the cytokine storm may have been repaired more compared to the direct use of the culture supernatant of dental pulp-derived stem cells.

### <Evaluation of Survival Rate in Mouse Model Lethally Damaged by LPS Administration (4)>

### (4) Comparison of Purified Microparticles and Stem Cells

A cytokine storm was induced in a mouse model, and the survival rate-improving effects of the cytokine storm suppressor of Example 1 containing dental pulp-derived stem cell exosomes, compared to those of the direct use of dental pulp-derived stem cells, were observed *in vivo* by the following method.

Five Slc:ICR mice were used per group.

Lipopolysaccharide (LPS), which is a cytokine storm inducer, was intraperitoneally administered to the mice at 30 mg/kg body weight.

Six hours after the LPS administration, 10 µg per mouse of the cytokine storm suppressor of Example 1 (purified by ultracentrifugation), 2×10⁶ cells per mouse of dental pulp-derived stem cells and 10 µg per mouse of physiological saline as a control were administered to the mice from the tail vein. The amount of the dental pulp-derived stem cells used (2×10⁶ cells per mouse) is the maximum number of the cell administration to a normal animal (the non-lethal maximum number) and is an adequate amount for comparative experiment.

The survival rates of the mice of the groups were observed every 12 hours from 12 hours after the LPS administration. The obtained results are shown in Fig. 5.

From Fig. 5, the cytokine storm suppressor of Example 1 containing dental pulp-derived stem cell exosomes was found to be able to significantly improve the survival rate when the cytokine storm suppressor was administered to an animal (mouse) with an induced cytokine storm compared to the direct use of dental pulp-derived stem cells and the control to which physiological saline was administered.

The improvement of the survival rate of the cytokine storm suppressor of Example 1 containing dental pulp-derived stem cell exosomes suggests that a tissue damaged by the cytokine storm may have been repaired more compared to the direct use of the dental pulp-derived stem cells.

### <Evaluation of Survival Rate in Mouse Model Lethally Damaged by LPS Administration (5)>

### (5) Comparison of Timings of Two Administrations

A cytokine storm was induced in a mouse model, and the survival rate-improving effects of the cytokine storm suppressor of Example 1 containing dental pulp-derived stem cell exosomes were observed *in vivo* by the following method for the purpose of comparing the influence of the timings of two administrations with that of the evaluation of the survival rate (3) while comparing with the direct use of a culture supernatant of dental pulp-derived stem cells.

Five Slc:ICR mice were used per group.

First, in the same manner as in the evaluation of the survival rate (3), LPS was administered to the mice, and 10 µg per mouse of the cytokine storm suppressor of Example 1 (purified by ultracentrifugation), 0.5 ml per mouse of a culture supernatant of dental pulp-derived stem cells and 10 µg per mouse of physiological saline as a control were administered six hours and 18 hours after the LPS administration. The survival rates of the mice of the groups were observed every 12 hours from 12 hours after the LPS administration. The obtained results are shown in Fig. 6.

Next, LPS was administered to the mice, and 10 µg per mouse of the cytokine storm suppressor of Example 1 (purified by ultracentrifugation), 0.5 ml per mouse of a culture supernatant of dental pulp-derived stem cells and 10 µg per mouse of physiological saline as a control were administered 0 hour (simultaneously with the LPS administration) and 12 hours after the LPS administration. The survival rates of the mice of the groups were observed every 12 hours from 12 hours after the LPS administration. The obtained results are shown in Fig. 7.

From Fig. 6 and Fig. 7, the cytokine storm suppressor of Example 1 containing dental pulp-derived stem cell exosomes was found to be able to significantly improve the survival rate further when the cytokine storm suppressor was administered to an animal (mouse) with an induced cytokine storm twice, namely simultaneously with the occurrence of the cytokine storm (LPS administration) (after 0 hour) and after 12 hours as in Fig. 7, compared to when the cytokine storm suppressor was administered twice, namely six hours and 18 hours after the occurrence of the cytokine storm (LPS administration) as in Fig. 6.

The improvement of the survival rate of the cytokine storm suppressor of Example 1 containing dental pulp-derived stem cell exosomes suggests that a tissue damaged by the cytokine storm may have been repaired more compared to the direct use of the culture supernatant of dental pulp-derived stem cells.

### <Repair of Damaged Tissues>

With respect to the mice used for the evaluation of the survival rate (1) to (5), the effects of the cytokine storm suppressors of the Examples containing exosomes of repairing the tissues of the whole body, especially all organs, which were damaged by the cytokine storm were observed based on the pictures of the tissues of all the organs of the mice, such as lungs and livers.

As a result, the cytokine storm suppressors of the Examples were found to be able to repair the tissues damaged by the cytokine storm when the cytokine storm suppressors were administered to an animal (mouse) with cytokine amounts increased by an induced cytokine storm compared to the control to which physiological saline was administered. In particular, the dental pulp-derived stem cell EVs (Example 1) were found to be able to significantly repair the lung tissue and the liver tissue damaged by the cytokine storm compared to the adipose-derived stem cell EVs (Example 2) and the bone marrow-derived stem cell EVs (Example 3).

The repair of the damaged tissues suggests that an aftereffect of a cytokine storm is improved.

## Claims

1. A cytokine storm suppressor containing microparticles derived from a culture supernatant of dental pulp-derived stem cells, adipose-derived stem cells, bone marrow-derived stem cells, umbilical cord-derived stem cells or immortalized stem cells thereof,
wherein the microparticles contain SOD3 in an effective amount capable of repairing a tissue or a cell damaged by a cytokine storm or a higher amount, wherein the SOD3 is extracellular superoxide dismutase,
the cytokine storm suppressor is not the culture supernatant, does not contain the dental pulp-derived stem cells, the adipose-derived stem cells, the bone marrow-derived stem cells, the umbilical cord-derived stem cells or the immortalized stem cells thereof, does not contain MCP-1 and does not contain Siglec-9, and
the cytokine storm suppressor is used for administration to a human or a nonhuman animal with a cytokine storm before damage of a tissue is caused by the cytokine storm to suppress the tissue damage by the cytokine storm through suppression of the cytokine storm or
used for administration to a human or a nonhuman animal with damage of a tissue caused by a cytokine storm to repair the tissue damaged by the cytokine storm through suppression of the cytokine storm.

2. The cytokine storm suppressor according to claim 1, wherein the microparticles are exosomes.

3. The cytokine storm suppressor according to claim 1 or 2, wherein the microparticles are microparticles purified from the culture supernatant.

4. The cytokine storm suppressor according to any one of claims 1 to 3 which suppresses all of IL-2, IL-4, IL-6, IL-10, IL-17, IFNγ and TNFα in a cell or in blood.

5. The cytokine storm suppressor according to any one of claims 1 to 4 which increases the survival rate when the cytokine storm suppressor is administered to a human or a nonhuman animal in a cytokine storm state compared to when the cytokine storm suppressor is not administered.

6. The cytokine storm suppressor according to any one of claims 1 to 5, wherein the microparticles contain 1.0 ng/mg or more of the SOD3 per 1 mg of the microparticles.

7. The cytokine storm suppressor according to any one of claims 1 to 6 which has a SOD3 activity of 0.3 unit/µg or more.

8. The cytokine storm suppressor according to any one of claims 1 to 7 which contains 0.01×10⁸ particles/ml or more of the microparticles.

9. The cytokine storm suppressor according to any one of claims 1 to 7 which contains 2.0×10⁹ particles/ml or more of the microparticles.

10. The cytokine storm suppressor according to any one of claims 1 to 9, wherein the dental pulp-derived stem cells, the adipose-derived stem cells, the bone marrow-derived stem cells or the umbilical cord-derived stem cells are human dental pulp-derived stem cells, human adipose-derived stem cells, human bone marrow-derived stem cells or human umbilical cord-derived stem cells.

11. The cytokine storm suppressor according to any one of claims 1 to 10, wherein the microparticles are microparticles derived from a culture supernatant of the dental pulp-derived stem cells.

12. The cytokine storm suppressor according to any one of claims 1 to 11, wherein the tissue damaged by the cytokine storm is a lung tissue.

13. The cytokine storm suppressor according to any one of claims 1 to 12, wherein the dental pulp-derived stem cells, the adipose-derived stem cells, the bone marrow-derived stem cells or the umbilical cord-derived stem cells are genetically modified cells in which the microparticles highly express the SOD3.

14. The cytokine storm suppressor according to any one of claims 1 to 13 which contains at least one of an anti-IL-6 antibody and a steroid.

15. The cytokine storm suppressor according to any one of claims 1 to 14 which is used for administration to a human or a nonhuman animal with a cytokine storm before damage of a tissue is caused by the cytokine storm to suppress the tissue damage by the cytokine storm through suppression of the cytokine storm and repair a tissue to be damaged by the cytokine storm.

16. The cytokine storm suppressor according to any one of claims 1 to 15, wherein the cytokine storm suppressor is a tissue-repairing agent.

17. A method for using a cytokine storm suppressor
which includes administering the cytokine storm suppressor according to any one of claims 1 to 16 to a human or a nonhuman animal
to decrease the cellular or blood cytokine amount of the human or the nonhuman animal and
which includes
a step of administering to the human or the nonhuman animal with a cytokine storm before damage of a tissue is caused by the cytokine storm to suppress the tissue damage by the cytokine storm through suppression of the cytokine storm or
a step of administering to the human or the nonhuman animal with damage of a tissue caused by a cytokine storm to repair the tissue damaged by the cytokine storm through suppression of the cytokine storm.

18. The method for using a cytokine storm suppressor according to claim 17, wherein the cytokine storm suppressor is used in combination with at least one of an anti-IL-6 antibody and a steroid.

19. A screening method for selecting a factor or a combination of factors which is effective for tissue repair for repairing a tissue damaged by a cytokine storm through suppression of the cytokine storm, having
a step of supplying one, two or more components contained in a culture supernatant obtained by culturing dental pulp-derived stem cells, adipose-derived stem cells, bone marrow-derived stem cells, umbilical cord-derived stem cells or immortalized stem cells thereof to an evaluation system related to SOD3, wherein the SOD3 is extracellular superoxide dismutase, and evaluating a SOD3-related feature.

20. A cytokine storm suppressor containing a component obtained by evaluation of the SOD3-related feature by the screening method according to claim 19,
wherein the component is derived from a culture supernatant of dental pulp-derived stem cells, adipose-derived stem cells, bone marrow-derived stem cells, umbilical cord-derived stem cells or immortalized stem cells thereof,
the component contains SOD3 in an effective amount capable of repairing a tissue or a cell damaged by a cytokine storm or a higher amount, wherein the SOD3 is extracellular superoxide dismutase,
the cytokine storm suppressor is not the culture supernatant, does not contain the dental pulp-derived stem cells, the adipose-derived stem cells, the bone marrow-derived stem cells, the umbilical cord-derived stem cells or the immortalized stem cells thereof, does not contain MCP-1 and does not contain Siglec-9,
the component satisfies at least one of the feature that 1.0 ng/mg or more of the SOD3 is contained per 1 mg of the component and the feature that the SOD3 activity is 0.3 unit/µg or more, and
the cytokine storm suppressor is used for administration to a human or a nonhuman animal with a cytokine storm before damage of a tissue is caused by the cytokine storm to suppress the tissue damage by the cytokine storm through suppression of the cytokine storm or
used for administration to a human or a nonhuman animal with damage of a tissue caused by a cytokine storm to repair the tissue damaged by the cytokine storm through suppression of the cytokine storm.
